(19) Europäisches Patentamt
European Patent Office
Office européen des brevets

(11) EP 0 893 433 A1

(12) EUROPEAN PATENT APPLICATION
published in accordance with Art. 158(3) EPC

(43) Date of publication:
**27.01.1999 Bulletin 1999/04**

(21) Application number: **97914602.4**

(22) Date of filing: **04.04.1997**

(51) Int. Cl.[6]: **C07C 251/50**, C07C 251/62, C07C 251/74, C07C 251/88, C07C 255/63, C07D 233/61, C07D 249/08, C07D 261/08, C07D 271/10, C07D 277/38, C07D 307/52

(86) International application number:
**PCT/JP97/01163**

(87) International publication number:
**WO 97/37968 (16.10.1997 Gazette 1997/44)**

(84) Designated Contracting States:
**AT BE CH DE DK ES FI FR GB GR IE IT LI LU MC NL PT SE**

(30) Priority: **11.04.1996 JP 89118/96**

(71) Applicant: **SHIONOGI & CO., LTD.**
**Osaka-shi, Osaka 541-0045 (JP)**

(72) Inventors:
- **KAI, Hiroyuki**
  **Yamatokoriyama-shi, Nara 639-11 (JP)**
- **MASUI, Moriyasu**
  **Yokkaichi-shi, Mi e 510-12 (JP)**

(74) Representative:
**Baverstock, Michael George Douglas et al**
**BOULT WADE TENNANT,**
**27 Furnival Street**
**London EC4A 1PQ (GB)**

# (54) ALPHA-ALKOXYIMINOBENZYL DERIVATIVES AND AGRICULTURAL CHEMICALS CONTAINING THEM AS ACTIVE INGREDIENTS

(57) A compound represented by the formula(I):

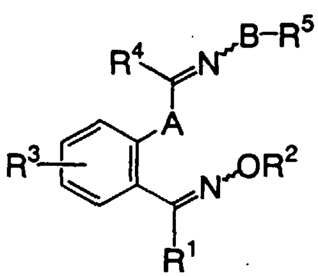

I

wherein $R^1$ is a heterocyclic group, CHO or $CH_2OR_6$; $R^2$ is an alkyl group; $R^3$ is hydrogen atom, an alkyl group, an alkoxy group, a halogen atom, nitro, cyano or a halogen-substituted alkyl group; $R^4$ is hydrogen atom, an alkyl group, a cycloalkyl group, an alkoxyalkyl group, an alkoxycarbonyl group, cyano, a halogen-substituted alkyl group, an aryl group or a heterocyclic group; $R^5$ is hydrogen atom, an alkyl group, a cycloalkyl group, an alkenyl group, an alkynyl group, an alkanoyl group, an aroyl group, an alkoxycarbonyl group, an aryl group or a heterocyclic group; A is (1) -$CH_2ON=C(R^7)$-, (2) -$CH_2N(R^8)$-$N=C(R^7)$-, (3) -$CH=NOC(R^9)(R^{10})$- or (4)-$CH=NN=C(R^7)$-; B is oxygen atom or $NR^{11}$; the wary line: ~ indicates E isomer, Z isomer or a mixture of them, has a broad spectrum of fungicidal activity, and therefore, is excellent as fungicides.

EP 0 893 433 A1

## Description

### Technical Field

The present invention relates to novel α-alkoxyiminobenzyl derivatives and agricultural fungicides containing the same as an active ingredient.

### Background Art

As α-alkoxyiminobenzyl derivatives, benzohydroxymoylazole derivatives (JP-A-1-308260, JP-A-5-1046, JP-A-5-331011, JP-A-5-331012, JP-A-6-41086, JP-A-6-145151, JP-A-6-345738, JP-A-7-173155 and WO92/09581) to have an insecticidal activity, oxime derivatives (JP-A-3-68559) to have an insecticidal activity, 1-azolyl-substituted oxime ether derivatives (JP-A-60-87269) to have fungicidal and bactericidal activities, oxazolyl or oxadiazolyl derivatives (WO94/22844) to have a fungicidal activity, pyrimidine derivatives (JP-A-6-49039, JP-A-7-48359 and WO94/08975) to have fungicidal and herbicidal activities, bezaldoxime derivatives (JP-A-7-70081) to have fungicidal, herbicidal and growth regulating activities and oxime derivatives (WO95/26956) to have a fungicidal activity are ever known.

However, as for the o-substituents on the α-alkoxyiminobenzyl ring, any compounds having similar substituents to those of the present invention are not disclosed specifically in the inventions as described above.

### Disclosure of Invention

An object of the present invention is to provide compounds having a stronger fungicidal activity and the like.

As the result of an extensive study to achieve the object as described above, it has been found that a series of alkoxyiminobenzyl derivatives of the following formula show a strong fungicidal activity, and the present invention has been accomplished.

The present invention relates to the compound represented by the formula (I):

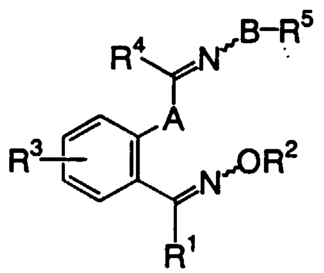

I

wherein $R^1$ is an optionally substituted heterocyclic group, CHO or $CH_2OR^6$ wherein $R^6$ is hydrogen atom, an alkyl group, an alkenyl group, an alkanoyl group, an alkoxycarbonyl group or an alkoxyalkyl group; $R^2$ is an alkyl group; $R^3$ is hydrogen atom, an alkyl group, an alkoxy group, a halogen atom, nitro, cyano or a halogen-substituted alkyl group; $R^4$ is hydrogen atom, an alkyl group, a cycloalkyl group, an alkoxyalkyl group, an alkoxycarbonyl group, cyano, a halogen-substituted alkyl group, an optionally substituted aryl group or an optionally substituted heterocyclic group; $R^5$ is hydrogen atom, an optionally substituted alkyl group, a cycloalkyl group, an alkenyl group, an alkynyl group, an alkanoyl group, an aroyl group, an alkoxycarbonyl group, an optionally substituted aryl group or an optionally substituted heterocyclic group; A is (1) $-CH_2ON=C(R^7)-$ wherein $R^7$ is hydrogen atom, an alkyl group, a halogen-substituted alkyl group, cyano, an alkylthio group or a cycloalkyl group, (2) - $CH_2N(R^8)-N=C(R^7)-$ wherein $R^8$ is hydrogen atom, an alkyl group or an alkanoyl group and $R^7$ is the same as defined above, (3) $-CH=NOC(R^9)(R^{10})-$ wherein $R^9$ and $R^{10}$ are each independently hydrogen atom, an alkyl group, cyano or a halogen-substituted alkyl group or (4) $-CH=NN=C(R^7)-$ wherein $R^7$ is the same as defined above; B is oxygen atom or $NR^{11}$ wherein $R^{11}$ is hydrogen atom, an alkyl group, phenyl or an alkanoyl group; the wavy line: ~ indicates E isomer, Z isomer or a mixture of them.

In this specification, the term "lower", unless otherwise mentioned, means a group having 1 to 8 carbon atoms, preferably 1 to 6 carbon atoms, particularly preferably 1 to 4 carbon atoms.

The optionally substituted heterocyclic group represented by the symbol $R^1$ means an unsubstituted or substituted heterocyclic group. The heterocyclic group includes, for example, a 5- to 7-membered heterocyclic group having 1 to 4 hetero atoms selected from the group consisting of nitrogen, sulfur and oxygen, specifically pyridyl (e.g. pyridin-2-yl and pyridin-3-yl), pyrimidinyl (e.g. pyrimidin-2-yl and pyrimidin-4-yl), isoxazolyl (e.g. isoxazol-3-yl, isoxazol-4-yl and isoxazol-5-yl), isoxazolinyl (e.g. 2-isoxazolin-3-yl and 2-isoxazolin-5-yl), isothiazolyl (e.g. isothiazol-5-yl), thiadiazolyl [e.g. 1,3,4-thiadiazolyl (e.g. 1,3,4-thiadiazol-2-yl) and 1,2,3-thiadiazolyl], pyridadinyl (e.g. pyridadin-2-yl), pyrazolyl (e.g. pyrazol-1-yl and pyrazol-5-yl), furyl (e.g. furan-2-yl), thienyl (e.g. thiophen-2-yl), imidazolyl (e.g. imidazol-1-yl and imidazol-2-yl), oxazolyl (e.g. oxazol-2-yl and oxazol-5-yl), thiazolyl (e.g. thiazol-2-yl), thiazolidinyl (e.g. thiazolidin-2-yl), oxadiazolyl [e.g. 1,3,4-oxadiazolyl (e.g. 1,3,4-oxadiazol-2-yl) and 1,2,4-oxadiazolyl], triazolyl [e.g. 1,2,4-triazolyl (e.g. 1H-1,2,4-triazol- 1-yl, 4H- 1,2,4-triazol-4-yl and 1,2,4-triazol-5-yl)], pyrazinyl, and the like.

Said heterocyclic group may form a condensed ring with a carbocyclic group or other heterocyclic group(s). Examples of the condensed ring are particularly benzoxazolyl (e.g. benzoxazol-2-yl), benzothiazolyl (e.g. benzothiazol-2-yl), benzoisoxazolyl (e.g. benzoisoxazol-3-yl), tetramethyleneisoxazolyl (e.g. 3,4-tetramethyleneisoxazol-5-yl and 4,5-tetramethyleneisoxazol-3-yl), and the like.

The heterocyclic group and the condensed ring may extend a bond or bonds at any possible position(s).

The substituent(s) of the heterocyclic group represented by the symbol $R^1$ is a lower alkyl group (e.g. methyl, ethyl, propyl and butyl), a lower alkenyl group (e.g. vinyl, allyl and 2-butenyl), a lower alkynyl group (e.g. ethynyl, 2-propynyl and 3-butynyl), a cycloalkyl group (e.g. cyclopropyl, cyclopentyl and cyclohexyl), a cycloalkenyl group (e.g. cyclopentenyl and cyclohexenyl), a halogen-substituted lower alkyl group (e.g. trifluoromethyl, trichloromethyl, difluoromethyl, chloromethyl, 2-bromoethyl and 1,2-dichloropropyl), a halogen atom (e.g. fluorine, chlorine, bromine and iodine), nitro, cyano, a lower alkylthio group (e.g. methylthio, ethylthio and propylthio), a group: $-OR^{12}$ wherein $R^{12}$ is hydrogen atom, a lower alkyl group (e.g. methyl, ethyl and propyl), a lower alkenyl group (e.g. vinyl, allyl and 2-butenyl), a lower alkynyl group (e.g. ethynyl, 2-propynyl and 3-butynyl), a lower alkylsulfonyl group (e.g. mesyl), phenyl, phenyl(lower)alkyl (e.g. benzyl and phenethyl), phenyl(lower)alkenyl (e.g. styryl and cinnamyl), and the like. A preferred substituent(s) among the above is a lower alkyl group or a halogen-substituted lower alkyl group, and preferably, methyl.

They may be at any position(s) on the heterocyclic ring and the preferred number of the above substituent(s) is 1-3.

The alkyl group represented by the symbol $R^6$ can cover, for example, C1 to C6 alkyl, preferably C 1 to C4 alkyl, particularly, methyl, ethyl, propyl, isopropyl, butyl, isobutyl, sec-butyl, tert-butyl and the like, especially methyl.

The alkenyl group represented by the symbol $R^6$ can cover, for example, C2 to C6 alkenyl, preferably C2 to C4 alkenyl, particularly, 1-propenyl, isopropenyl, allyl, 2-butenyl and the like, especially allyl.

The alkanoyl group represented by the symbol $R^6$ can cover, for example, an alkanoyl group having C1 to C6 alkyl, preferably, C1 to C4 alkyl, specifically for instance, acetyl, trifluoroacetyl, propionyl, butyryl, and the like. Specifically acetyl is preferable.

The alkoxycarbonyl group represented by the symbol $R^6$ can cover, for example, an alkoxycarbonyl group having C1 to C6 alkyl, preferably, C1 to C4 alkyl, specifically for instance, methoxycarbonyl, ethoxycarbonyl, propoxycarbonyl and the like. Specifically methoxycarbonyl is preferable.

The alkoxyalkyl group represented by the symbol $R^6$ can cover, for example, C2 to C8 alkoxyalkyl, preferably, C2 to C5 alkoxyalkyl, specifically for instance, methoxymethyl, methoxyethyl, methoxypropyl, ethoxymethyl, ethoxyethyl, propoxymethyl, propoxyethyl and the like. Specifically methoxymethyl and ethoxymethyl are preferable.

The preferred $R^1$ is isoxazol-3-yl, isoxazol-4-yl, isoxazol-5-yl, 2-isoxazolin-3-yl, 2-isoxazolin-5-yl, imidazol-1-yl, imidazol-2-yl, 1,3,4-oxadiazol-2-yl, furan-2-yl, 1,2,4-oxadiazol-3-yl, 1H-1,2,4-triazole-1-yl, thiazolidin-2-yl, oxazol-5-yl, each of which may optionally be substituted CHO or $CH_2OR^6$.

The preferred $R^1$ is specifically imidazol-1-yl, 1-methylimidazol-2-yl, isoxazol-3-yl, 3-methylisoxazol-5-yl, 5-methylisoxazol-3-yl, 2-isoxazolin-3-yl, 1,3,4-oxadiazol-2-yl, CHO, $CH_2OH$ or $CH_2OCH_3$.

Examples of the alkyl group represented by the symbol $R^2$ are the same as those of the alkyl group represented by the above symbol $R^6$. Specifically methyl is preferable.

Examples of the alkyl group represented by the symbol $R^3$ are the same as those of the alkyl group represented by the above symbol $R^6$.

The alkoxy group represented by the symbol $R^3$ can cover, for example, C1 to C6 alkoxy, preferably C1 to C4 alkoxy, particularly, methoxy, ethoxy, propoxy, isopropoxy, butoxy, isobutoxy, sec-butoxy, tert-butoxy, and the like.

Examples of the halogen atom represented by the symbol $R^3$ are fluorine, chlorine, bromine and iodine.

The halogen-substituted alkyl group represented by the symbol $R^3$ can cover, for example, C1 to C6 alkyl, preferably, C1 to C4 alkyl, specifically for instance, an alkyl group (e.g. methyl, ethyl- propyl, isopropyl and butyl) substituted by at least one of halogen atoms (e.g. fluorine, chlorine, bromine and iodine). Examples of the above halogen-substituted alkyl group are difluoromethyl, trifluoromethyl, trichloromethyl, 1,2-dichloropropyl, and the like.

The preferred $R^3$ is hydrogen.

Examples of the alkyl group represented by the symbol $R^4$ are the same as those of the alkyl group represented by the above symbol $R^6$.

The cycloalkyl group represented by the symbol $R^4$ can cover, for example, C3 to C8 cycloalkyl, preferably C3 to C6 cycloalkyl, specifically cyclopropyl, cyclopentyl, cyclohexyl, and the like.

Examples of the alkoxyalkyl group represented by the symbol $R^4$ are the same as those of the alkoxyalkyl group represented by the above symbol $R^6$.

Examples of the alkoxycarbonyl group represented by the symbol $R^4$ are the same as those of the alkoxycarbonyl group represented by the above symbol $R^6$.

Examples of the halogen-substituted alkyl group represented by the symbol $R^4$ are the same as those of the halogen-substituted alkyl group represented by the above symbol $R^3$.

The aryl part of the optionally substituted aryl group represented by the symbol $R^4$ can cover, for example, C6 to C14 aryl, specifically phenyl, naphtyl (1-naphtyl, 2-naphtyl), and the like.

The optionally substituted heterocyclic group represented by the symbol $R^4$ means an unsubstituted or substituted heterocyclic group. The heterocyclic group includes, for example, a 5- to 7- membered heterocyclic group having 1 to 4 hetero atoms selected from the group consisting of nitrogen, sulfur and oxygen, specifically pyridyl (e.g. pyridin-2-yl, pyridin-3-yl and pyridin-4-yl), pyrimidinyl (e.g. pyrimidin-2-yl, pyrimidin-4-yl and pyrimidin-5-yl), isoxazolyl (e.g. isoxazol-3-yl and isoxazol-5-yl), thiadiazolyl (e.g. 1,3,4-thiadiazol-2-yl and 1,2,3-thiadiazolyl), pyridazinyl (e.g. pyridazin-3-yl), pyrrolyl, pyrazolyl, furyl (e.g. furan-2-yl and furan-3-yl), thienyl (e.g. thiophen-2-yl and thiophen-3-yl), imidazolyl, oxazolyl, thiazolyl (e.g. thiazol-2-yl), oxadiazolyl (e.g. 1,3,4-oxadiazol-2-yl and 1,2,4-oxadiazol-5-yl), pyrazinyl, quinolyl (e.g. quinolin-2-yl), indolyl, and the like. The heterocyclic group may form a condensed ring with a carbocyclic group or another heterocyclic group.

The substituents of the substituted aryl group and of the substituted heterocyclic group represented by the symbol $R^4$ are the same as those of the substituted heterocyclic group represented by the above symbol $R^1$. Specifically, a halogen atom, a lower alkyl group, a halogen-substituted lower alkyl group, a lower alkoxy group and a lower alkylthio group are preferable. They may be at any position(s) on the heterocyclic ring and the preferred number of the above substituent(s) is 1-3.

The preferred $R^4$ is hydrogen atom, methyl, ethyl, methoxycarbonyl, ethoxycarbonyl, cyano or an optionally substituted phenyl group.

Examples of the alkyl of the optionally substituted alkyl group represented by the symbol $R^5$ are the same as those of the alkyl group represented by the above symbol $R^6$. The substituents of the above alkyl group represented by the symbol $R^5$ are cyano, a haogen atom (e.g. fluorine, chlorine, bromine and iodine), a cycloalkyl group (e.g. cyclopropyl, cyclobutyl, cyclopentyl and cyclohexyl), an alkoxy group (e.g. methoxy, ethoxy, propoxy and butoxy), an alkanoyl group (e.g. acetyl, propionyl, butyryl and trifluoroacetyl), an alkoxycarbonyl (e.g. methoxycarbonyl, ethoxycarbonyl and propoxycarbonyl), an optionally substituted aryl group (e.g. 1-naphtyl, 2-naphtyl and phenyl), an optionally substituted heterocyclic group (e.g. the optionally substituted heterocyclic group represented by the above symbol $R^4$) and an optionally substituted carbamoyl group (e.g. unsubstituted and mono- or di-substituted carbamoyl, wherein examples of the substituent(s) of the mono- or di-substituted carbamoyl are the same as those of the substituted heterocyclic group represented by the above symbol $R^1$; The two substituents of the disubstituted carbamoyl may be the same or different from each other and may form, taken together, a monocyclic group or a polycyclic group. The monocyclic group and the polycyclic group can cover a 4 to 8 membered cyclic group optionally having a hetero atom (e.g. oxygen, nitrogen and sulfur), which is formed by the two substituents and the adjacent nitrogen atom. The monocyclic group and the polycyclic group may be condensed with another ring to form a condensed polycyclic group. Examples of the monocyclic group and the polycyclic group include pyrrolidine, piperidine, morpholine, thiomorpholine, piperazine, hexamethyleneimine, tetrahydroquinoline, tetrahydroisoquinoline, and the like. Examples of the substituent(s) of the monocyclic group and the polycyclic group are the same as those of the substituted heterocyclic group represented by the above symbol $R^1$.), and the like.

Examples of the cycloalkyl group represented by the above symbol $R^5$ are the same as those of the cycloalkyl group represented by the above symbol $R^4$.

Examples of the alkenyl group represented by the above symbol $R^5$ are the same as those of the alkenyl group represented by the above symbol $R^6$.

The alkynyl group represented by the symbol $R^5$ can cover, for example, C3 to C6 alkynyl, preferably C3 to C4 alkynyl, particularly, 2-propynyl, 3-butynyl and the like.

Examples of the alkanoyl group represented by the above symbol $R^5$ are the same as those of the alkanoyl group represented by the above symbol $R^6$.

The aroyl group represented by the symbol $R^5$ can cover, for example, C7 to C15 aroyl, particularly, benzoyl, naphtoyl and the like.

Examples of the alkoxycarbonyl group represented by the above symbol $R^5$ are the same as those of the alkoxycarbonyl group represented by the above symbol $R^6$.

Examples of the optionally substituted aryl group represented by the above symbol $R^5$ are the same as those of the optionally substituted aryl group represented by the above symbol $R^4$.

Examples of the optionally substituted heterocyclic group represented by the above symbol $R^5$ are the same as those of the optionally substituted heterocyclic group represented by the above symbol $R^4$.

The preferred $R^5$ is hydrogen atom, methyl, ethyl, propyl, isopropyl, butyl, allyl, 2-propynyl, methoxymethyl, ethoxymethyl, acetyl, methoxycarbonyl, ethoxycarbonyl or an optionally substituted benzyl.

Examples of the alkyl group represented by the symbols $R^7$ to $R^{10}$ are the same as those of the alkyl group represented by the symbol $R^6$.

Examples of the halogen-substituted alkyl group represented by the symbols $R^7$, $R^9$ and $R^{10}$ are the same as those of the halogen-substituted alkyl group by the above symbol $R^3$.

The alkylthio group represented by the symbol $R^7$ can cover, for example, C1 to C6 alkylthio, preferably C1 to C3 alkylthio, particularly, methylthio, ethylthio, propylthio and the like.

Examples of the cycloalkyl group represented by the symbol $R^7$ are the same as those of the cycloalkyl group represented by the symbol $R^4$.

Examples of the alkanoyl group represented by the symbol $R^8$ are the same as those of the alkanoyl group represented by the symbol $R^6$.

The preferred A is a group: $-CH_2ON{=}C(CH_3)-$, $-CH_2ON{=}C(C_2H_5)-$,$-CH_2ON{=}C(CN)-$, $-CH_2N(Ac)N{=}C(CH_3)-$, $-CH{=}NOCH(CH_3)-$, $-CH{=}NOCH(C_2H_5)-$, $-CH{=}NOC(CH_3)_2-$, $-CH{=}NN{=}C(CH_3)-$ or $-CH{=}NN{=}C(C_2H_5)-$.

The preferred A is a group: $-CH_2ON{=}C(CH_3)-$, $-CH_2ON{=}C(C_2H_5)-$, $-CH_2ON{=}C(CN)-$, $-CH_2N(Ac)N{=}C(CH_3)-$, $-CH{=}NOCH(CH_3)-$, $-CH{=}NOCH(C_2H_5)-$, $-CH{=}NOC(CH_3)_2-$, $-CH{=}NN{=}C(CH_3)-$ or $-CH{=}NN{=}C(C_2H_5)-$.

Examples of the alkyl group represented by the symbol $R^{11}$ are the same as those of the alkyl group represented by the symbol $R^6$.

Examples of the alkanoyl group represented by the symbol $R^{11}$ are the same as those of the alkanoyl group represented by the symbol $R^6$.

The preferred B is oxygen atom or a group: $-NH-$, $-N(CH_3)-$, $-N(Ac)-$ or $-N(Ph)-$.

There are two isomers i.e. E and Z isomers responsible for the imino structure in the compound of the present invention, and the present invention includes each of the isomers and a mixture of them in any ratio. In this specification, it is indicated by a wavy line: ~ in the general formula.

Best Mode for Carrying Out the Invention

The compound (I) of the present invention (namely the compound represented by the formula (I): the compound represented by the other formula may be referred to in the same way.) can be produced, for example, according to the following preparation routes.

Route 1

Reaction scheme 1

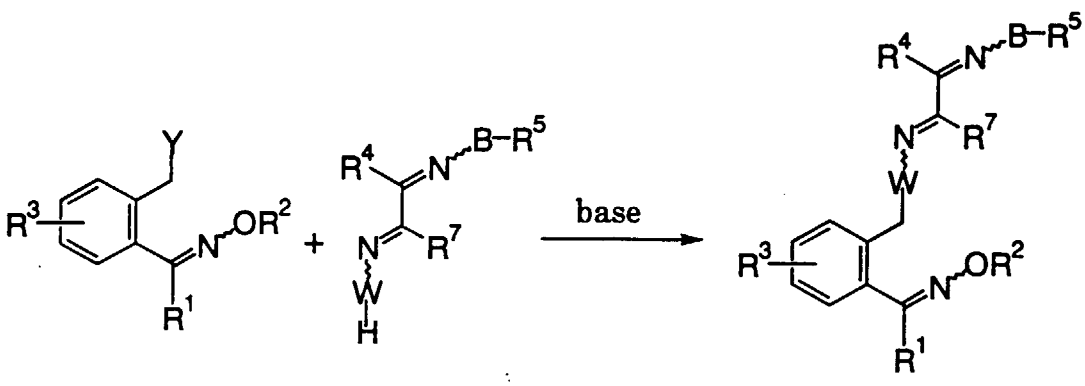


II III Ia

wherein Y is a halogen atom (e.g. chlorine, bromine and iodine), W is oxygen atom or $NR^8$ and the other symbol are the same as defined above.

Namely, the compound (II) may be reacted with the compound (III) in the presence of a base in a suitable solvent (alone or a mixture) or without any solvent to give the compound represented by the general formula (Ia).

The amount of the compound (III) to be used is 1 or more equivalents, preferably 1 to 2 equivalents to the compound (II) in this reaction.

Examples of the base to be used include hydroxides of metal (e.g. sodium hydroxide and potassium hydroxide), carbonates of metal (e.g. sodium carbonate and potassium carbonate), alkoxides of metal (e.g. sodium methoxide,

sodium ethoxide and potassium tert-butoxide), hydrides of metal (e.g. sodium hydride and potassium hydride), and the amount of these bases to be used is 1 or more equivalents, preferably 1 to 3 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), ethers (e.g. tetrahydrofuran and dioxane), ketones (e.g. acetone and methyl ethyl ketone), nitriles (e.g. acetonitrile), water and their mixture.

The reaction temperature is set at -30 °C to 160 °C, preferably -10 °C to 120 °C. The reaction time is usually about 0.5 to 120 hours, varing with the properties of the compound.

The compound (Ia) may be purified, if necessary, by a conventional method (e.g. column chromatography and recrystallization).

Further, the compound (II) to be used as starting material in this reaction can be produced by the method described in WO95/26956 and the compound (III) can be produced by the method described in WO95/18789.

Route 2

Reaction scheme 2

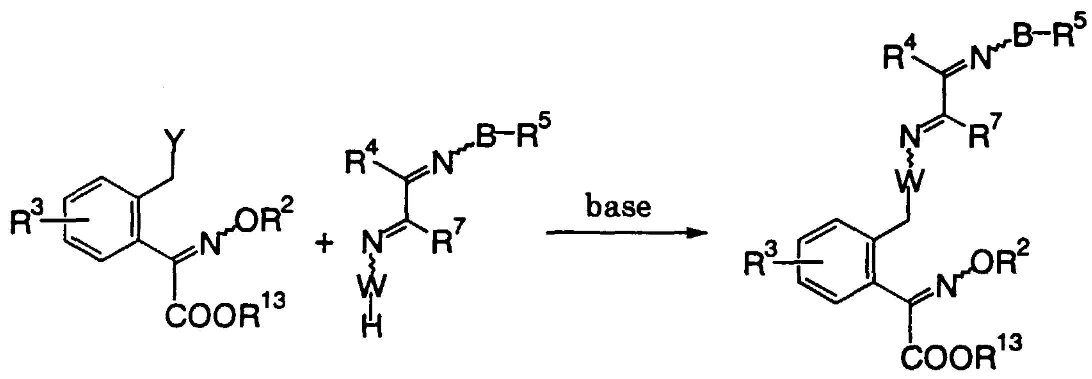


IV III V

wherein $R^{13}$ is an alkyl group (e.g. methyl and ethyl) and the other symbols are the same as defined above.

Namely, the compound (IV) may be reacted with the compound (III) in the presence of a base in a suitable solvent (alone or a mixture) or without any solvent to give the compound represented by the general formula (V).

The amount of the compound (III) to be used is 1 or more equivalents, preferably 1 to 2 equivalents to the compound (IV) in this reaction.

Examples of the base and the solvent to be used are the same as those used in the above reaction scheme 1.

The reaction temperature is set at -30 °C to 150 °C, preferably -10 °C to 100 °C. The reaction time is usually about 0.5 to 120 hours, varing with the properties of the compound.

The compound (V) may be used in the subsequent step as the reaction mixture or the crude product, or after purification by a conventional method (e.g. column chromatography and recrystallization).

Further, the compound (IV) to be used as starting material in this reaction can be produced by the method described in JP-A 3-246268 or JP-A 5-97768.

Reaction scheme 3

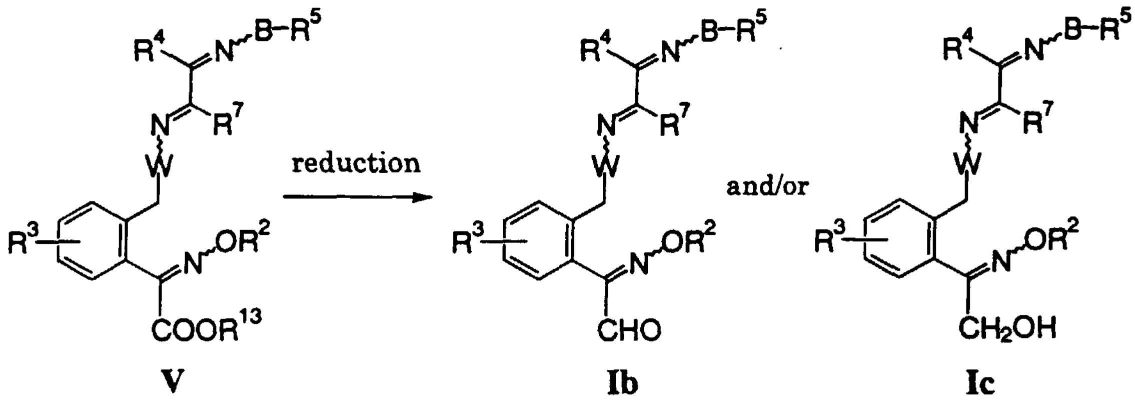

wherein each symbol is the same as defined above.

Namely, the compound of the present invention represented by the general formula (Ib) may be produced by reducing the compound (V) with various kinds of hydride of metals.

Examples of the hydride of metals to be used are lithium aluminum hydride, potassium aluminum hydride, sodium aluminum hydride, sodium diisobutylaluminum hydride, diisobutylaluminum hydride, sodium bis(methoxyethoxy)aluminum hydride, bis(N-methylpiperazino)aluminum hydride, lithium trimethoxyaluminum hydride, lithium triethoxyaluminum hydride, and the like.

The compound represented by the general formula (Ic) may be produced by reducing the compound (V) with various kinds of hydride of metals or by the catalytic hydrogenation method.

Examples of the hydride of metals to be used are aluminum hydride, lithium aluminum hydride, potassium aluminum hydride-aluminum chloride, lithium trimethoxyaluminum hydride, sodium bis(methoxyethoxy)aluminum hydride, diisobutylaluminum hydride, sodium borohydride, sodium aluminum hydride, lithium tritert-butoxyaluminum hydride, magnesium aluminum hydride, lithium borohydride, and the like.

Examples of the catalyst to be used for the catalytic hydrogenation are copper cromite, zinc cromite, rhenium oxide, palladium, Ranynickel, and the like.

There is justly a difference between the reduction process for the compound (Ib) and that for the compound (Ic). Further, the structure of the compound (V) differentiates the reduction process. Examples of the difference on the reduction process are, for instance, kinds and equivalents of the hydride of metals and the reaction condition (e.g. temperature and time).

The production of the compound (Ib) and the compound (Ic) by the reduction process of the compound (V) can be carried out by a conventional method, for instance, the method detailed in M. Hudlicky "Reduction in Organic Chemistry" Ellis Horwood Limited(1984) and the like.

If the compound (Ib) and the compound (Ic) are given as a mixture, they may be used in the subsequent step as the crude mixture or, if necessary, as a mixture after purification by a conventional method (e.g. column chromatography and recrystallization), or, if necessary, the compound (Ib) and the compound (Ic) may be separated.

Route 3

Reaction scheme 4

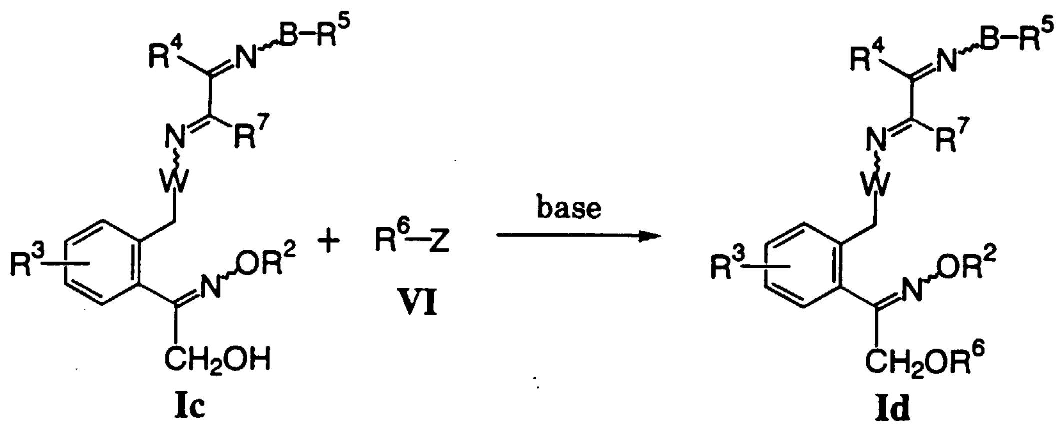


wherein Z is a halogen atom (e.g. chlorine and bromine), an alkylsulfonyloxy group (e.g. a lower alkylsulfonyloxy group (e.g. methylsulfonyloxy and ethylsulfonyloxy)), an alkoxysulfonyloxy group (e.g. a lower alkoxysulfonyloxy group (e.g. methoxysulfonyloxy and ethoxysulfonyloxy)) and an arylsulfonyloxy group (e.g. benzenesulfonyloxy and p-toluenesulfonyloxy) and the other symbols are the same as defined above.

Namely, the compound (Ic) may be reacted with the compound (VI) in the presence of a base in a suitable solvent (alone or a mixture) to give the compound represented by the general formula (Id).

The amount of the compound (VI) to be used is 1 or more equivalents, preferably 1 to 2 equivalents to the compound (Ic) in this reaction.

Examples of the base to be used include, depending on the properties of the compound(VI), hydrides of metal (e.g. sodium hydride and potassium hydride), carbonates of metal (e.g. sodium carbonate and potassium carbonate), alkoxides of metal (e.g. sodium methoxide, sodium ethoxide and potassium tert-butoxide), organic bases (e.g. pyridine and triethylamine) and the amount of these bases to be used is 1 or more equivalents, preferably 1 to 2 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), halogen substituted hydrocarbons (e.g. dichloromethane and 1,2-dichloroethane), ethers (e.g. tetrahydrofuran and dioxane), nitriles (e.g. acetonitrile), water and their mixture.

The reaction temperature is set at -30 °C to 150 °C, preferably -10 °C to 100 °C.

The reaction time is usually about 0.5 to 90 hours, varing with the properties of the compound.

The compound (Id) may be purified, if necessary, by a conventional method (e.g. column chromatography and recrystallization).

Route 4

Reaction scheme 5

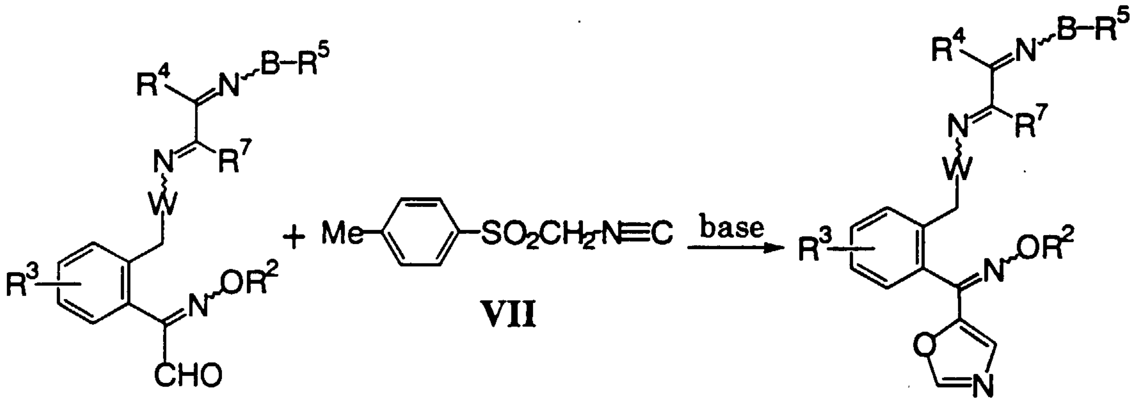


Ib

VII

Ie

wherein each symbol is the same as defined above.

Namely, the compound (Ib) may be reacted with the compound (VII) in the presence of a base in a suitable solvent (alone or a mixture), as described in JP-A-58-131984, to give the compound represented by the general formula (Ie).

The amount of the compound (VII) to be used is 1 or more equivalents, preferably 1 to 2 equivalents to the compound (Ib) in this reaction.

Examples of the base to be used include hydroxides of metal (e.g. sodium hydroxide and potassium hydroxide), carbonates of metal (e.g. sodium carbonate and potassium carbonate), alkoxides of metal (e.g. sodium methoxide, sodium ethoxide and potassium tert-butoxide), and the amount of these bases to be used is 1 or more equivalents, preferably 1 to 2 equivalents.

Examples of the solvent to be used are aromatic hydrocarbons (e.g. toluene, benzene and xylene), saturated hydrocarbons (e.g. cyclohexane and hexane), alcohols (e.g methanol, ethanol and propanol), and their mixture.

The reaction temperature is set at 30 °C to 150 °C,preferably 50 °C to 100 °C. The reaction time is usually about 0.5 to 90 hours, varing with the properties of the compound.

The compound (Ie) may be purified, if necessary, by a conventional method (e.g. column chromatography and recrystallization).

Route 5

Reaction scheme 6

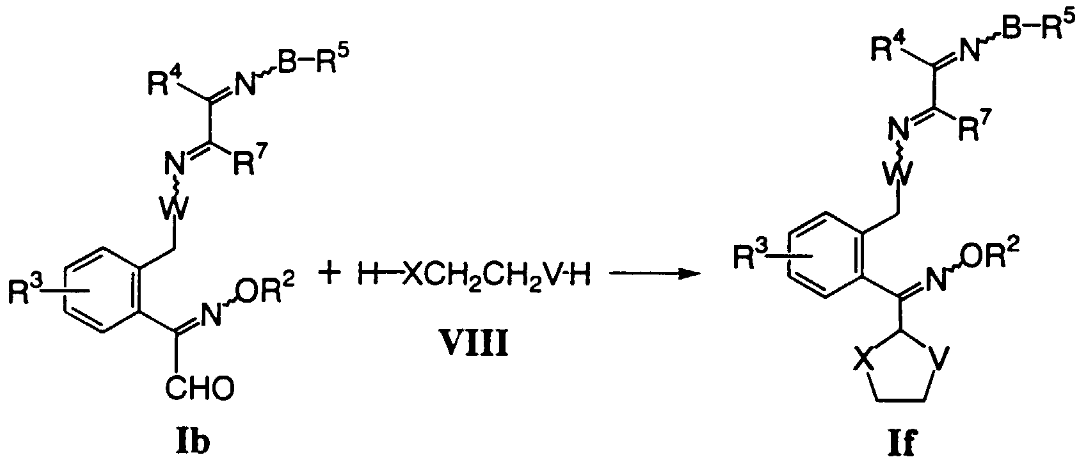

wherein X and V are each independently oxygen atom, sulfur atom or N-$R^{14}$ wherein $R^{14}$ is hydrogen atom or a lower alkyl group, and the other symbols are the same as defined above.

Namely, the compound (Ib) may be reacted with the compound (VIII) or its salt (e.g. hydrochloride and hydrobromide) in the presence or the absence of a base, an acid or a salt of metal in a suitable solvent (alone or a mixture) or without any solvent as described in T. W. Green, "Protective Groups in Organic Synthesis", p.109 to 151, John Willy & Sons (1981) to give the compound of the present invention represented by the general formula (If).

The amount of the compound (VIII) to be used is 1 or more equivalents, preferably 1 to 5 equivalents to the compound (Ib) in this reaction.

Examples of the base to be used include amines (e.g. triethylamine), and the amount of these bases to be used is 1 or more equivalents, preferably 1 to 6 equivalents to the compound (Ib).

Examples of the acid to be used include inorganic acids (e.g. hydrochloric acid and sulfuric acid) and sulfonic acids (e.g. p-toluenesulfonic acid), and the amount of these acids to be used is 0.01 to 0.5 equivalents, preferably 0.02 to 0.2 equivalents to the compound (Ib).

Examples of the salt of metal to be used are potassium carbonate, zinc acetate and the like, and the amount of these salts to be used is 0.01 to 0.5 equivalents, preferably 0.02 to 0.2 equivalents to the compound (Ib).

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), halogen substituted hydrocarbons (e.g. dichloromethane and 1,2-dichloroethane), ethers (e.g. tetrahydrofuran and dioxane), alcohols (e.g. butanol, 2-methoxyethanol, ethlene glycol and glycerol), and a mixture of them.

The reaction temperature is set at 20 °C to 200 °C,preferably 50 °C to 160 °C.

The reaction time is usually about 0.5 to 90 hours, varing with the properties of the compound.

The compound (If) may be purified, if necessary, by a conventional method (e.g. column chromatography and recrystallization).

Route 6

Reaction scheme 7

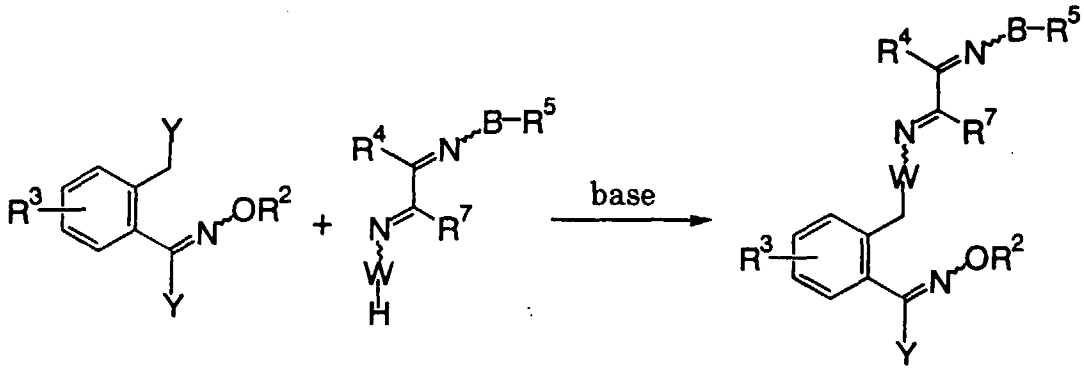


IX III X

wherein each symbol is the same as defined above.

Namely, the compound (IX) may be reacted with the compound (III) in the presence of a base in a suitable solvent (alone or a mixture) or without any solvent to give the compound represented by the general formula (X).

The amount of the compound (III) to be used is 1 or more equivalents, preferably 1 to 2 equivalents to the compound (IX) in this reaction.

Examples of the base to be used include hydroxides of metal (e.g. sodium hydroxide and potassium hydroxide), carbonates of metal (e.g. sodium carbonate and potassium carbonate), alkoxides of metal (e.g. sodium methoxide, sodium ethoxide and potassium tert-butoxide), hydrides of metal (e.g. sodium hydride and potassium hydride), and the amount of these bases to be used is 1 or more equivalents, preferably 1 to 3 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), ethers (e.g. tetrahydrofuran and dioxane), ketones (e.g. acetone and methyl ethyl ketone), nitriles (e.g. acetonitrile), water and a mixture of them.

The reaction temperature is set at -30 °C to 160 °C, preferably - 10 °C to 120 °C. The reaction time is usually about 0.5 to 120 hours, varing with the properties of the compound.

The compound (X) may be used in the subsequent step as the reaction mixture or the crude product, or, if necessary, after purification by a conventional method (e.g. column chromatography and recrystallization).

Further, the compound (IX) to be used as starting material in this reaction can be produced by the method described in WO 95/26956.

Reaction Scheme 8

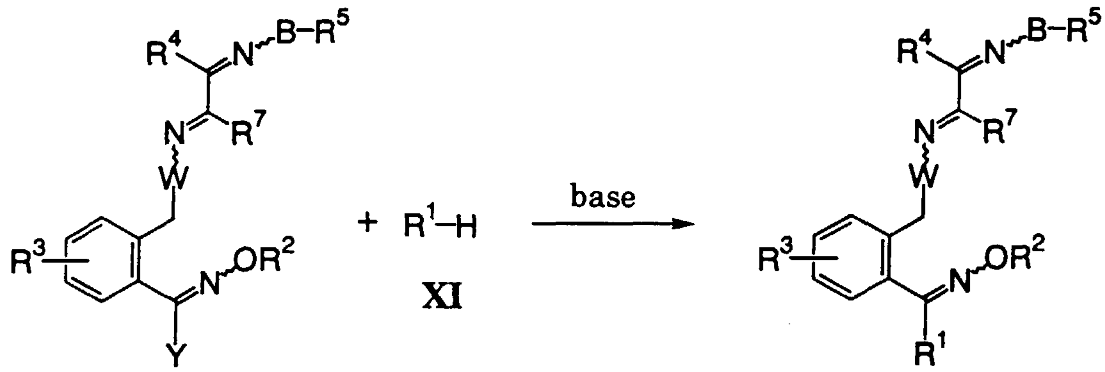


XI

X Ia

wherein each symbol is the same as defined above. In this reaction, the preferred $R^1$ is an optionally substituted imidazolyl group (e.g. imidazol-1-yl) or an optionally substituted triazolyl group (e.g. 1H-1,2,4-triazol-1-yl).

The compound (X) may be reacted with the compound (XI) in the presence or absence of a base in a suitable solvent (alone or a mixture) or without any solvent to give the compound of the present invention represented by the general formula (Ia).

The amount of the compound (XI)to be used is 1 or more equivalents, preferably 1 to 5 equivalents to the compound (X)in this reaction.

Examples of the base to be used include hydroxides of metal (e.g. sodium hydroxide and potassium hydroxide), hydrides of metal (e.g. sodium hydride and potassium hydride), carbonates of metal (e.g. sodium carbonate and potassium carbonate), alkoxides of metal (e.g. sodium methoxide, sodium ethoxide and potassium tert-butoxide), amines (e.g. pyridine, triethylamine), and the amount of these bases to be used is 1 or more equivalents, preferably 1 to 5 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), halogen substituted hydrocarbons (e.g. dichloromethane and 1,2-dichloroethane), ethers (e.g. tetrahydrofuran and dioxane), ketones (e.g. acetone and methyl ethyl ketone), nitriles (e.g. acetonitrile), water and a mixture of them.

The reaction temperature is set at -30 °C to 170 °C, preferably -10 °C to 140 °C. The reaction time is usually about 0.5 to 100 hours, varing with the properties of the compound.

The compound (Ia) may be purified, if necessary, by a conventional method (e.g. column chromatography and recrystallization).

Route 7

Reaction Scheme 9

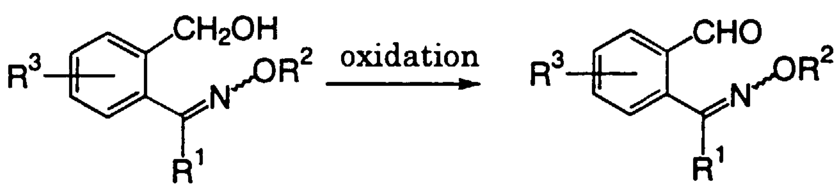


XII XIII

wherein each symbol is the same as defined above.

Namely, the compound (XII) may be oxidized by various kinds of a oxidizing agents to give a benzaldehyde derivative represented by the general formula (XIII).

Examples of the oxidizing agent to be used are pyridinium chlorochromate, tert-butyl chromate, nikel peroxide, activated dimethylsulfoxide, and the like.

Various kinds of the activated dimethylsulfoxide oxidation method using activated dimethylsulfoxide have been known and if Swern oxidation is used, the amounts of dimethylsulfoxide and oxalyl chloride as an electrophilic agent are each 1 or more equivalents, preferably 1 to 4 equivalents to the compound (XII).

Examples of the solvent to be used are halogen-substituted hydrocarbons (e.g. dichloromethane).

The reaction temperature is set at -78 to -20 °C, preferably -78 to -40 °C. The reaction time is 15 minutes to 3 hours, varing with the properties of the compound.

Next, a base such as triethylamine may be added to form a sulfonium ylide.

The amount of the base to be used is 1 or more equivalents, 1 to 6 equivalents to the compound (XII).

The reaction temperature is set at -78 to 50 °C, preferably -78 to 30 °C. The reaction time is 15 minutes to 3 hours, varing with the properties of the compound.

After the completion of the reaction, water is added, and the product is extracted with the solvent.

The compound (XIII) may be used in the subsequent step as the reaction mixture or the crude product, or after purification by a conventional method (e.g. column chromatography and recrystallization).

Further, the compound (XII) to be used as starting material in this reaction can be produced by the method, for instance, described in WO95/26956.

Reaction Scheme 10

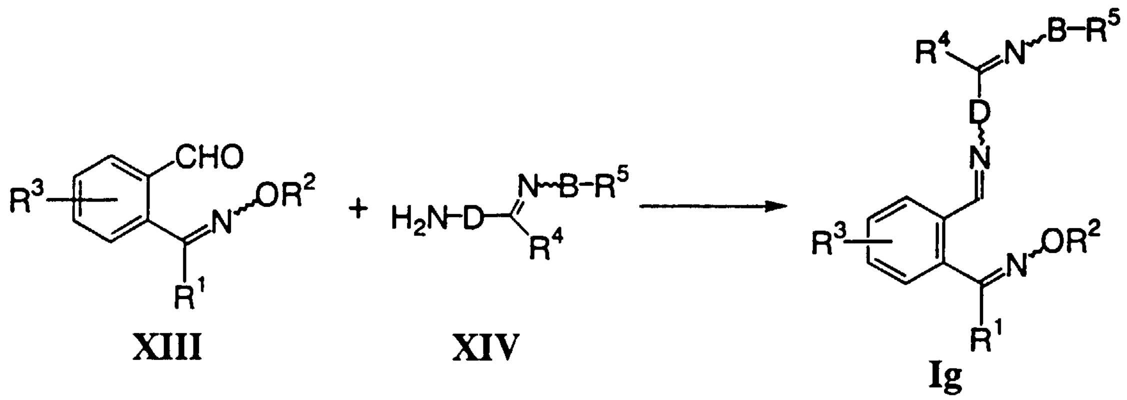

XIII XIV Ig

wherein D is $-OC(R^9)(R^{10})-$ or $-N=C(R^7)-$ and the other symbols are the same as defined above.

Namely, the compound (XII) may be reacted with an O-substituted hydroxylamine or a hydrazone derivative or their salt (e.g. hydrochloride and sulfate) represented by the formula (XIV) in a suitable solvent (alone or a mixture) to produce the compound of the present invention represented by the general formula (Ig).

In this reaction, the amount of the compound (XIV) is 1 or more equivalents, preferably 1 to 3 equivalents to the compound (XIII).

Examples of the solvent to be used are aromatic hydrocarbons (e.g. toluene, benzene and xylene), saturated hydrocarbons (e.g. cyclohexane and hexane), alcohols (e.g. methanol, ethanol and propanol), ethers (e.g. tetrahydrofuran and dioxane), water and a mixture of them.

The reaction temperature is set at 0 to 160 °C, preferably 20 to 130 °C. The reaction time is 0.5 to 90 hours, varing with the properties of the compound.

If necessary, the compound (Ig) may be purified by a conventional method (e.g. column chromatography and recrystallization).

Further, the hydrazone derivative represented by the compound (XIV) to be used as starting material in this reaction can be produced by the reaction of the corresponding ketone derivatives with and hydrazine.

The O-substituted hydroxylamine derivative represented by the compound (XIV) can be produced by the known method or a similar tthat to the method described in Houben-Weyl, "Methoden der Organischen Chemie", X/1.

Route 8

Reaction Scheme 11

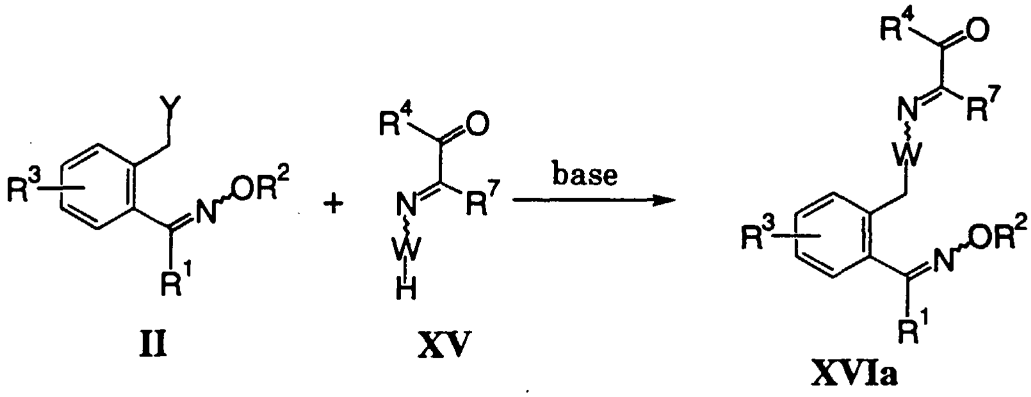


II XV XVIa

wherein each symbol is the same as defined above.

Namely, the compound (II) may be reacted with the compound (XV) in the presence of a base in a suitable solvent (alone or a mixture) or without any solvent to produce the compound represented by the general formula (XVIa).

The amount of the compound (XV) to be used is 1 or more equivalents, preferably 1 to 2 equivalents to the compound (II).

Examples of the base and the solvent to be used are the same as those and the solvent to be used in the above reaction scheme 1.

The reaction temperature is set at -30 °C to 150 °C, preferably -10 °C to 100 °C. The reaction time is usually about 0.5 hours to 120 hours.

The compound (XVIa) may be to be used in the subsequent step as the reaction mixture or the crude product, or after purification by a conventional method (e.g. column chromatography and recrystallization).

Further, the compound (XV), to be used as starting material ion may be used as commercially available or produced by the known method.

Reaction scheme 12

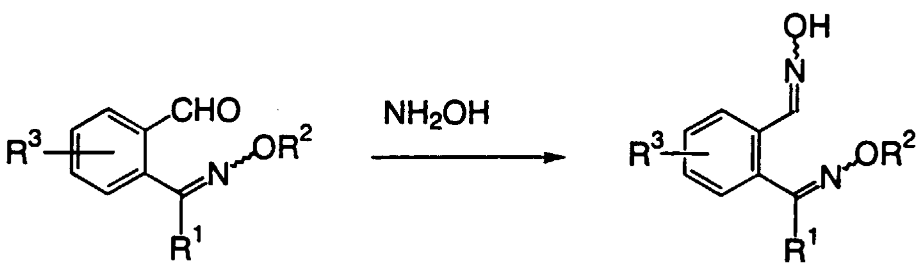


XIII XVII

wherein each symbol is the same as defined above.

Namely, the compound (XIII) may be reacted with hydroxylamine or its salt in a suitable solvent to give the compound (XVII).

The amount of hydroxylamine to be used is 1 to 4 equivalents, preferably 1 to 2.5 equivalents to the compound (XIII).

Examples of the salt of hydroxylamine are salts of mineral acid (e.g. hydrochloride and sulfate) and the like. The salt may be used after neutralization by a base. Examples of the base to be used are hydroxides of metal (e.g. sodium

hydroxide and potassium hydroxide), carbonates of metal (e.g. sodium carbonate and potassium carbonate), alkoxides of metal (e.g. sodium methoxide and sodium ethoxide), pyridine and the like, and the amount of the base to be used is 1 to 3 equivalents, preferably 1 to 2 equivalents to a salt of hydoxylamine.

Examples of the solvent to be used are hydrocarbons (e.g. benzene, toluene and xylene), halogen-substituted hydrocarbons (e.g. chloroform and 1,2-dichloroethane), ethers (e.g. tetrahydrofuran and dioxane), alcohols (e.g. methanol, ethanol, n-propanol and isopropanol), water and a mixture of them.

The reaction temperature is set at 0 to 150 °C, preferably 20 to 100 °C. The reaction time is usually about 15 minutes to 24 hours.

The compound (XVII) may be used in the subsequent step as the reaction mixture or the crude product, or after purification by a conventional method (e.g. column chromatography and recrystallization).

Reaction Scheme 13

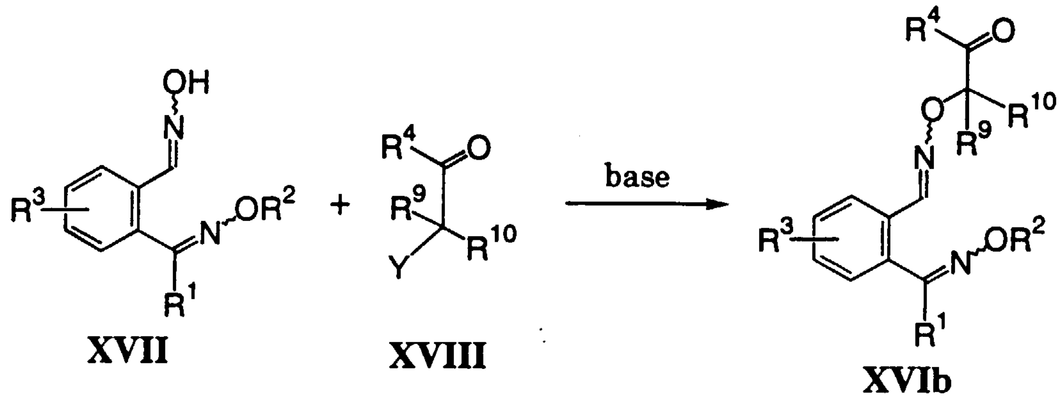


XVII XVIII XVIb

wherein each symbol is the same as defined above.

The compound (XVII) may be reacted with the compound (XVIII) in the presence of a base in a suitable solvent which may be used alone or as a mixture or in the absence of a solvent to produce the compound represented by the general formula (XVIb).

In this reaction, the amount of the compound (XVIII) to be used is 1 or more equivalents to the compound (XVII).

Examples of the base to be used are hydroxides of metal (e.g. sodium hydroxide and potassium hydroxide), carbonates of metal (e.g. sodium carbonate and potassium carbonate), alkoxides of metal (e.g. sodium methoxide, sodium ethoxide and potassium tert-butoxide), hydrides of metal (e.g. sodium hydride and potassium hydride), and the like, and the amount of the base to be used is 1 or more equivalents, preferably 1 to 3 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), ethers (e.g. tetrahydrofuran and dioxane), ketones (e.g. acetone and methyl ethyl ketone), nitriles (e.g. acetonitrile), water, the mixture of them, and the like.

The reaction temperature is set at -30 to 120 °C, preferably 0 to 90 °C. The reaction time is 0.5 to 90 hours, varing with the properties of the compound.

The compound (XVIb) may be used in the subsequent step as the reaction mixture or the crude product, or after purification by a conventional method (e.g. column chromatography and recrystallization).

Reaction Scheme 14

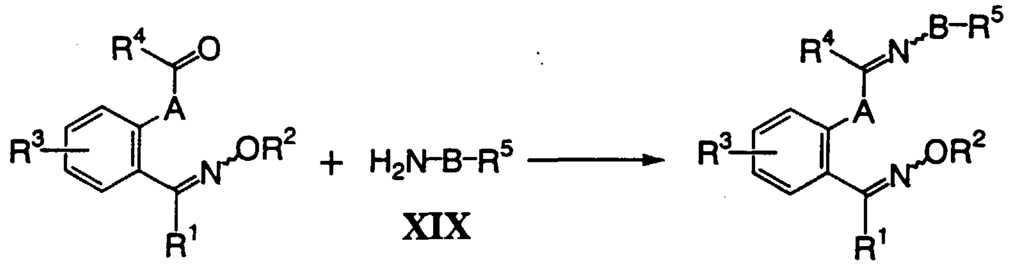

XVI XIX I

wherein each symbol is the same as defined above.

Namely, the compound (XVI) may be reacted with an O-substituted hydroxylamine or a hydrazine derivative and their salt (e.g. hydrochloride and sulfate) represented by the compound (XIX) in a suitable solvent (alone or a mixture) to produce the compound of the present invention represented by the general formula (I)-

The salt may be used after neutralization by a base. Examples of the base to be used are hydroxides of metal (e.g. sodium hydroxide and potassium hydroxide), carbonates of metal (e.g. sodium carbonate and potassium carbonate), alkoxides of metal (e.g. sodium methoxide and sodium ethoxide), pyridine and the like, and the amount of the base to be used is 1 to 3 equivalents, preferably 1 to 2 equivalents to the salt of the compound (XIX).

In this reaction, the amount of the compound (XIX) is 1 or more equivalents, preferably 1 to 3 equivalents to the compound (XVI).

Examples of the solvent to be used are aromatic hydrocarbons (e.g. toluene, benzene and xylene), saturated hydrocarbons (e.g. cyclohexane and hexane), alcohols (e.g. methanol, ethanol and propanol), ethers (e.g. tetrahydrofuran and dioxane), water and a mixture of them.

The reaction temperature is set at 0 to 160 °C, preferably 20 to 130 °C. The reaction time is 0.5 to 90 hours, varing with the properties of the compound.

If necessary, the compound (I) may be purified by a conventional method (e.g. column chromatography and recrystallization).

The O-substituted hydroxylamine derivative represented by the compound (XIX) can be produced by the known method or a similar method to that described in Houben-Weyl, "Methoden der Organischen Chemie", X/1.

The hydrazine derivative represented by the compound (XIX) can be produced by the known method or a similar method to that described in Houben-Weyl, "Methoden der Organischen Chemie", X/2.

Reaction scheme 15

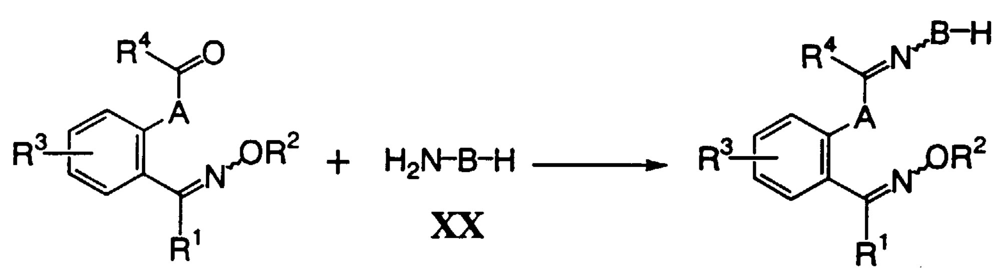

XVI XX XXI

wherein each symbol is the same as defined above.

Namely, the compound (XVI) may be reacted with a hydroxylamine or a hydrazine and their salt (e.g. hydrochloride and sulfate) represented by the compound (XX) in a suitable solvent (alone or a mixture) to produce the compound represented by the general formula (XXI).

The salt may be used after neutralization by a base. Examples of the base to be used are hydroxides of metal (e.g.

sodium hydroxide and potassium hydroxide), carbonates of metal (e.g. sodium carbonate and potassium carbonate), alkoxides of metal (e.g. sodium methoxide and sodium ethoxide), pyridine and the like, and the amount of the base to be used is 1 to 3 equivalents, preferably 1 to 2 equivalents to the salt of the compound (XX).

In this reaction, the amount of the compound (XX) to be used is 1 or more equivalents, preferably 1 to 3 equivalents to the compound (XVI).

Examples of the solvent to be used are aromatic hydrocarbons (e.g. toluene, benzene and xylene), saturated hydrocarbons (e.g. cyclohexane and hexane), alcohols (e.g. methanol, ethanol and propanol), ethers (tetrahydrofuran and dioxane), water and a mixture of them.

The reaction temperature is set at 0 to 160 °C, preferably 20 to 130 °C. The reaction time is usually about 0.5 to 90 hours, varing with the properties of the compound.

The compound (XXI) may be used in the subsequent step as the reaction mixture or the crude product, or after purification by a conventional method (e.g. column chromatography and recrystallization).

Reaction scheme 16

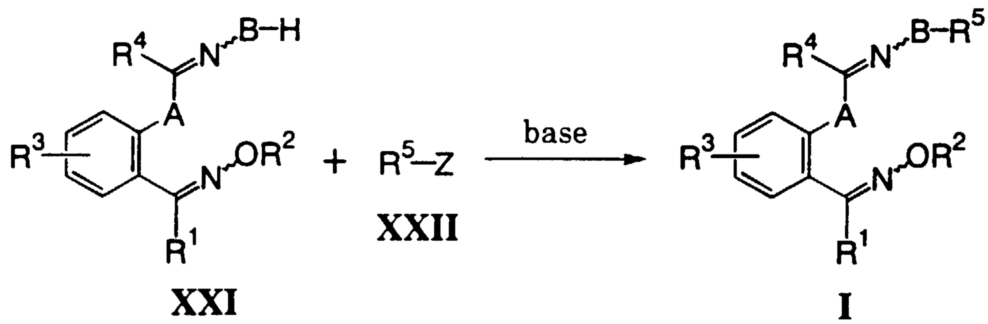


wherein each symbol is the same as define above.

Namely, the compound (XXI) may be reacted with the compound (XXII) in the presence of a base in a suitable solvent (alone or a mixture) to produce the compound of the present invention represented by the general formula (I).

In this reaction, the compound (XXII) to be used is 1 or more equivalents, eferably 1 to 2 equivalents to the compound (XXI).

Examples of the base to be used are, depending on the properties of the compound (XXII), hydrides of metal (e.g. sodium hydride and potassium hydride), carbonates of metal (e.g. sodium carbonate and potassium carbonate), alkoxides of metal (e.g. sodium methoxide, sodium ethoxide and potassium tert-butoxide), organic bases (e.g. pyridine and triethylamine) and the like, and the amount of the base to be used is 1 or more equivalents, preferably 1 to 2 equivalents.

Examples of the solvent to be used are N,N-dimethylformamide (DMF), dimethylsulfoxide (DMSO), aromatic hydrocarbons (e.g. toluene, benzene and xylene), saturated hydrocarbons (e.g. cyclohexane and hexane), halogen substituted hydrocarbons (e.g. dichloromethane and 1,2-dichloroethane), ethers (tetrahydrofuran and dioxane), nitriles (e.g. acetonitriles), water and a mixture of them.

The reaction temperature is set at -30 to 150 °C, preferably -10 to 100 °C.

The reaction time is usually about 0.5 to 90 hours, varing with the properties of the compound.

The compound (I) may be purified, if necessary, by a conventional method (e.g. column chromatography and recrystallization).

Route 9

Reaction Scheme 17

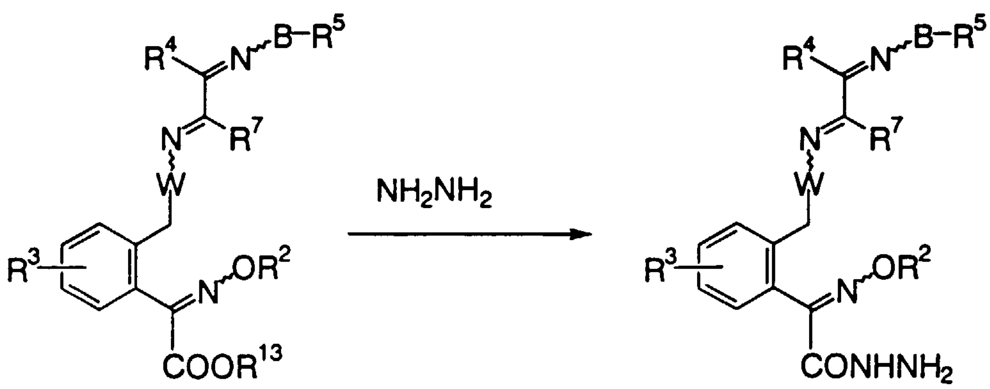


V XXIII

wherein each symbol is the same as defined above.

Namely, the compound (V) may be reacted with hydrazine mono hydrate or their salt (e.g. hydrochloride and sulfate) in a suitable solvent (alone or a mixture) to produce the compound represented by the general formula (XIII).

In this reaction, the amount of hydrazine to be used is 1 or more equivalents, preferably 1 to 5 equivalents to the compound (V).

Examples of the solvent to be used are aromatic hydrocarbons (e.g. toluene, benzene and xylene), saturated hydrocarbons (e.g. cyclohexane and hexane), alcohols (e.g. methanol, ethanol and propanol), ethers (e.g. THF and dioxane), water and a mixture of them.

The reaction temperature is set at 0 °C to 160 °C, preferably 10 °C to 130 °C. The reaction time is 0.5 to 90 hours, varing with the properties of the compound.

The compound (XXIII) may be used in the subsequent step as the reaction mixture or the crude product, or after purification by a conventional method (e.g. column chromatography and recrystallization).

Reaction Scheme 18

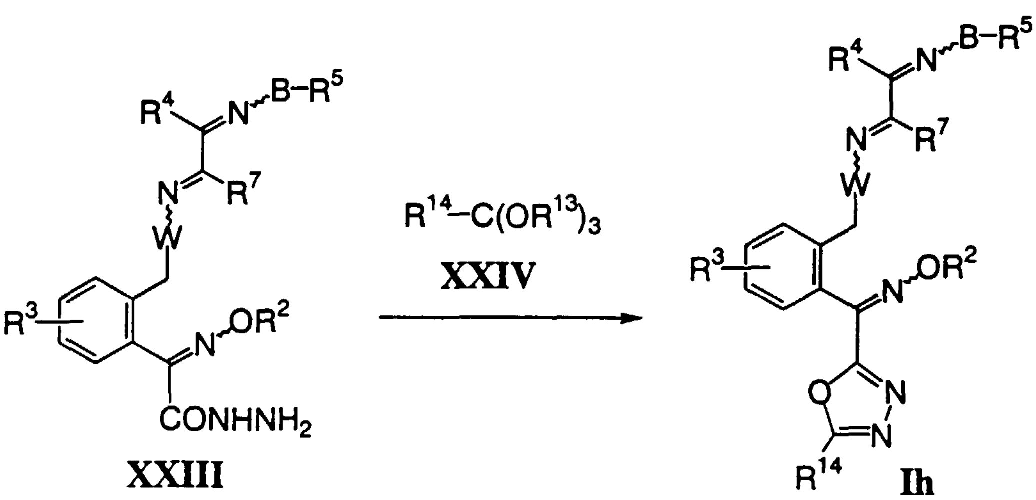


XXIII Ih

wherein each symbol is the same as defined above.

Namely, the compound (XXIII) may be reacted with the compound (XXIV) in the presence or absence of an acid in a suitable solvents (alone or a mixture) or without any solvent, as described in C. Ainaworth, J. Am. Chem. Soc. 77,1148 (1955) to produce the compound of the present invention represented by the general formula (Ih).

In this reaction, the amount of the compound (XXIV) to be used is 1 or more equivalents, preferably 1 to 20 equivalents to the compound (XXIII).

Examples of the acids to be used are halogenated hydrogens (e.g. hydrogen chloride, hydrogen bromide and hydrogen iodide), sulfonic acids (e.g. aliphatic sulfonic acid (e.g. trifluoromethanesulfonic acid) and aromatic sulfonic acids (e.g. toluenesulfonic acid)), and the like.

The amount of the acid to be used is a trace to 1 equivalent to the compound (XXIII).

Examples of the solvent to be used are aromatic hydrocarbons (e.g. toluene, benzene and xylene), saturated hydrocarbons (e.g. cyclohexane and hexane), ethers (e.g. tetrahydrofurane and dioxane) and a mixture of them.

The reaction temperature is set at 20 to 200 °C, preferably 50 to 170 °C. The reaction time is 0.5 to 90 hours, varing with the properties of the compound.

The compound (Ih) may be purified, if necessary, by a conventional method (e.g. column chromatography and recrystallization).

The compounds (I) of the present invention show a strong fungicidal activity against a wide variety of phytopathogenic fungi on crop plants or their seeds (e.g. rice plant, wheat, barley, rye, corn, common millet, millet, buck wheat, soybean, redbean and peanut), vegetables (e.g. cucumber, eggplant, tomato, pumpkin and kidney bean), fruit trees (e.g. citrus fruits, grape, apple, pear and peach), etc. They also show a fungicidal activity against phytopathogenic fungi in soil. Examples of the phytopathogenic fungi on which the compounds of this invention exert their fungicidal activity are Pyricularia oryzae, Rhizoctonia solani, Erysiphe graminis, Sphaerotheca fuliginea, Erysiphe cichoracearum, Phylophthora infestans, Pseudoperonospora cubensis, Peronospora manshurica, Plasaopara viticola, Botrytis cinerea of vegetable, grape, and the like, Pythium aphanidermatum, Sclerotinia sclerotiorum, Corticium rolfsii of buckwheat, soybean, redbean, colza and so on, Pseudocercosporella herpotrichoides, etc. Therefore, the compounds (I) of the present invention are useful as fungicides, particularly agricultural fungicides.

The compounds (I) of the present invention may be applied to plants by any conventional procedures such as atomizing, scattering or spreading. They may be also applied through treatment of seeds of plants, soil where plants grow, soil for seeding, paddy field or water for perfusion with the active compound. Application may be performed before or after the infection with phytopathogenic fungi on plants.

For the practical usage, the compounds (I) of the present invention may be applied as such or in a formulation form such as solutions, wettable powders, emulsions, suspensions, concentrated liquid preparations, tablets, granules, aerosols, powders, pastes, fumigants, flowable, etc. which is suitable for agricultural fungicides.

Such formulation form can be prepared in a conventional manner by mixing at least one of the compounds (I) of the present invention with an appropriate solid or liquid carrier (s) and, if necessary, an appropriate adjuvant (s) (e.g. surfactants, spreaders, dispersants and stabilizers) for improving the dispersibility and other properties of the active ingredient.

Examples of the solid carriers or diluents are botanical materials (e.g. flour, tobacco stalk powder, soybean powder, walnut-shell powder, vegetable powder, saw dust, bran, bark powder, cellulose powder and vegetable extract residue), fibrous materials (e.g. paper, corrugated cardboard and old rags), artificial plastic powders, clays (e.g. kaolin, bentonite and fuller's earth), talcs, other inorganic materials (e.g. pyrophyllite, sericite, pumice, sulfur dust powder and active carbon), chemical fertilizers (e.g. ammonium sulfate, ammonium phosphate, ammonium nitrate, urea and ammonium chloride), and the like.

Examples of the liquid carriers or diluents are water, alcohols (e.g. methanol and ethanol), ketones (e.g. acetone and methyl ethyl ketone), ethers (e.g. diethyl ether, dioxane, cellosolve and tetrahydrofuran), aromatic hydrocarbons (e.g. benzene, toluene, xylene and methylnaphthalene), aliphatic hydrocarbons (e.g. gasoline, kerosene and lamp oil), esters, nitriles, acid amides (e.g. dimethylformamide and dimethylacetamide), halogen substituted hydrocarbons (e.g. dichloroethane and carbon tetrachloride), and the like.

Examples of the surfactants are alkyl sulfuric esters, alkyl sulfonates, alkyl aryl-sulfonates, polyethylene glycol ethers, polyhydric alcohol esters, and the like. Examples of the spreaders or dispersants may include casein, gelatin, starch powder, carboxymethyl cellulose, gum arabic, alginic acid, lignin, bentonite, molasses, polyvinyl alcohol, pine oil and agar. As the stabilizers, there may be used PAP (a mixture of isopropyl phosphate), tricresyl phosphate (TCP), tall oil, epoxydized oil, surfactants, fatty acids and their esters, etc.

The composition of the present invention may contain other fungicides, insecticides, herbicides or fertilizers in addition to the above ingredients.

In general the above composition contains at least one of the compounds (I) of the present invention in a concentration of 1 to 95 % by weight, preferably, 2.0 to 80 % by weight. The composition can be used as such or in a diluted form. About 1 g to 5 kg/hectare, preferably about 10 g to 1000 g/hectare, of the compound of the present invention is used in a concentration of normally about 1 to 5,000 ppm, preferably about 10 to 1,000 ppm.

Examples

Practical and presently preferred embodiments of the invention are illustratively shown in the following examples, but the present invention is not limited only to these Examples. $^1$H-NMR ($CDCl_3$) described in Examples were measured in deuterochloroform with 270 MHz spectrometer and are given in $\delta$ values (ppm) with tetramethylsilane as an internal standard. The coupling constants (J) are given in Hz. The abbreviations in the date have the following meaning; s : singlet, d : doublet, t : triplet, q : quartet, sept: septet, m : multiplet.

EXAMPLE 1

Synthesis of 3- [$\alpha$-methoxyimino-2-(2-methoxyimino-1-methylpropylideneaminooxymethyl)benzyl] isoxazole

To a solution of 0.23 g (1.0 mmol) of 3-(2-chloromethyl-$\alpha$-methoxyiminobenzyl)isoxazole, 3ml of DMF, 0.16 g (1.2mmol) of 3-methoxyimino-2-butanoneoxime and 0.05 g (1.2 mmol) of 60 % sodium hydride was added under ice cooling and the mixture was stirred at room temperature for 2 hours. To the reaction mixture, 100 ml of ether was added and the mixture was washed with 80 ml of brine twice. The ether layer was dried over anhydrous magnesium sulfate and concentrated under reduced pressure. The residue was purified by silica gel column chromatography with a mixture of ethyl acetate and n-hexane to give 0.19 g (yield 55.2 %) of 3-[$\alpha$-methoxyimino-2-(2-methoxyimino-1-methylpropylideneaminooxymethyl)benzyl]isoxazole as a colorless crystal.

mp 125 to 126.5 °C
$^1$H-NMR ($CDCl_3$) $\delta$ ppm 1.85 (3H, s), 1.90 (3H, s), 3.92 (3H, s), 4.00 (3H, s), 5.09 (2H, s), 6.74 (1H, d, J=1.8), 7.24-7.54 (4H, m), 8.38 (1H, d, J=1.8).

EXAMPLE 2

Synthesis of 2-methoxyimino-2-[2-(2-methoxyimino-1-methylpropylideneaminooxymethyl)phenyl]ethanol and 2-methoxyimino-2-[2-(2-methoxyimino-1-methylpropylideneaminooxymethyl)phenyl]acetamide

(1) Synthesis of methyl $\alpha$-methoxyimino-2-(2-methoxyimino-1-methylpropylideneaminooxymethyl)phenylacetate

To a solution of 2.86 g (10 mmol) of methyl 2-bromomethyl-$\alpha$-methoxyiminophenylacetate, 10 ml of DMF, 1.43 g (11 mmol) of 3-methoxyimino-2-butanoneoxime and 0.44 g (11 mmol) of 60 % sodium hydride was added under ice cooling and the mixture was stirred at room temperature for 3 hours. To the reaction mixture was added 200 ml of ether and the mixture was washed with 100 ml of brine twice. The ether layer was dried over anhydrous magnesium sulfate and concentrated under reduced pressure. The residue was purified by silica gel column chromatography with a mixture of ethyl acetate and n-hexane to give 2.62 g (yield 78.1 %) of methyl $\alpha$-methoxyimino-2-(2-methoxyimino-1-methylpropylideneaminooxymethyl)phenylacetate as a colorless crystal.

$^1$H-NMR($CDCl_3$) $\delta$ ppm: 1.95 (3H, s), 1.99 (3H, s), 3.84 (3H, s), 3.93 (3H, s), 4.04 (3H, s), 5.06 (2H, s), 7.15-7.20 (1H, m), 7.34-7.47 (3H, m).

(2) Synthesis of 2-methoxyimino-2-[2-(2-methoxyimino-1-methylpropylideneaminooxymethyl)phenyl]ethanol and 2-methoxyimino-2-[2-(2-methoxyimino-1-methylpropylideneaminooxymethyl)phenyl]acetaldehyde

To a mixture of 2.35 g (7 mmol) of methyl $\alpha$-methoxyimino-2-(2-methoxyimino-1-methylpropylideneaminooxymethyl)phenylacetate and 14 ml of dichloromethane, 8.4 ml (8.4 mmol) of 1M diisobutylaluminium hydride / toluene solution was added at - 60 °C for 10 minutes and the mixture was stirred at -78 °C to 0 °C for 5 hours. To the reaction mixture, 6 ml of methanol was added and the mixture was stirred at room temperature for an hour. The insoluble material was separated and concentrated under reduced pressure and the residue was purified by silica gel column chromatography with a mixture of ethyl acetate and n-hexane to give 0.77 g (yield 35.8 %) of (2-methoxyimino-2-[2-(2-methoxyimino-1-methylpropylideneaminooxymethyl)phenyl] ethanol and 0.77 g (yield 36.0 %) of 2-methoxyimino-2-[2-(2-methoxyimino-1-methylpropylideneaminooxymethyl)phenyl]acetaldehyde both as a colorless crystal.

alcohol : mp 84 to 85 °C
$^1$H-NMR ($CDCl_3$) $\delta$ ppm: 1.96 (3H, s), 2.03 (3H, s), 2.74 (1H, t, J=5.5), 3.87 (3H, s), 3.94 (3H, s), 4.43 (2H, d, J=5.5), 5.09 (2H, s), 7.12-7.18 (1H, m), 7.34-7.51 (3H, m).
aldehyde : mp 61 to 63 °C

$^1$H-NMR ($CDCl_3$) δ ppm: 1.93 (3H, s), 1.97 (3H, s), 3.93 (3H, s), 4.11 (3H, s), 5.01 (2H, s), 7.08-7.12 (1H, m), 7.34-7.47 (3H, m), 10.03 (1H, s).

EXAMPLE 3

Synthesis of 5-[α-methoxyimino-2-(2-methoxyimino-1-methylpropylideneaminooxymethyl)benzyl]oxazole

To 0.24 g (0.8 mmol) of 2-methoxyimino-2-[2-(2-methoxyimino-1-methylpropylideneaminooxymethyl)phenyl]acetaldehyde, 0.19 g (0.96 mmol) of p-toluenesulfonylmethylisocyanide, 0.14 g (1.04 mmol) of potassium carbonate and 3 ml of methanol was added and the mixture was refluxed for 3 hours with stirring. When the reaction was completed, 100 ml of ether was added and the mixture was washed with 80 ml of brine twice. The ether layer was dried over anhydrous magnesium sulfate and concentrated under reduced pressure. The residue was purified by silica gel column chromatography with a mixture of ethyl acetate and n-hexane, and was recrystallized from a mixture of ethyl acetate and n-hexane to give 0.11 g (yield 39.9 %) of 5-[α-methoxyimino-2-(2-methoxyimino-1-methylpropylidneaminooxymethyl)benzyl]oxazole as a colorless crystal.

mp 84 to 85 °C
$^1$H-NMR ($CDCl_3$) δ ppm: 1.86 (3H, s), 1.89 (3H, s), 3.92 (3H, s), 4.00 (3H, s), 5.09 (2H, s), 6.82 (1H, s), 7.21-7.54 (4H, m), 7.92 (1H, s).

EXAMPLE 4

Synthesis of 2-[α-methoxyimino-2-(2-methoxyimino-1-methylpropylideneaminooxymethyl)benzyl]thiazolidine

To 0.31 g (1 mmol) of 2-methoxyimino-2-[2-(2-methoxyimino-1-methylpropylideneaminooxymethyl)phenyl]acetaldehyde, 2 ml of toluene, 2 ml of buthanol, 0.23 g (2 mmol) of cysteamine hydrochloride and 0.28 ml (2 mmol) of triethylamine was added and the mixture was stirred at room temperature for 3 hours. When the reaction was completed, 50 ml of a half saturated brine was added and the mixture was extracted with 100 ml of ethyl acetate twice. The extract was dried over anhydrous magnesium sulfate and concentrated under reduced pressure. The residue was purified by silica gel column chromatography with a mixture of ethyl acetate and n-hexane to give 0.36 g (yield 98.8 %) of 2-[α-methoxyimino-2-(2-methoxyimino-1-methylpropylideneaminooxymethyl)benzyl]thiazolidine as a colorless crystal.

mp 83 to 84.5 °C
$^1$H-NMR ($CDCl_3$) δ ppm: 1.98 (3H, s), 2.07 (3H, s), 2.70-3.10 (3H, m), 3.40-3.70 (2H, m), 3.89 (3H, s), 3.94 (3H, s), 5.14-5.50 (3H, m), 7.30-7.51 (4H, m).

EXAMPLE 5

Synthesis of 1-[α-methoxyimino-2-(2-methoxyimino-1-methylpropylideneaminooxymethyl)benzyl]imidazole

(1) Synthesis of 2-(2-methoxyimino-1-methylpropylideneaminooxymethyl)-α-methoxyiminobenzyl chloride

To 1.09 g (5 mmol) of 2-chloromethyl-α-methoxyiminobenzyl chloride, 0.65 g (5 mmol) of 3-methoxyimino-2-butanoneoxime, 5 ml of DMF and 1.04 g (7.5 mmol) of potassium carbonate was added and the mixture was stirred at room temperature for 3 days. When the reaction was completed, 100 ml of ether was added and the mixture was washed with 100 ml of brine twice. The ether layer was dried over anhydrous magnesium sulfate and concentrated under reduced pressure. The residue was purified by silica gel column chromatography with a mixture of ethyl acetate and n-hexane to give 0.97 g (yield 91.2 %) of 2-(2-methoxyimino-1-methylpropylideneaminooxymethyl)-α-methoxyiminobenzyl chloride as a colorless oil.

$^1$H-NMR ($CDCl_3$) δ ppm: 1.97 (3H, s), 2.07 (3H, s), 3.94 (3H, s), 4.08 (3H, s), 5.41 (2H, s), 7.33-7.61 (4H, m).

(2) Synthesis of 1-[α-methoxyimino-2-(2-methoxyimino-1-methylpropylideneaminooxymethyl)benzyl]imidazole

To 0.27 g (3.9 mmol) of imidazole, 3 ml of dimethylformamide and 0.16 g (3.9 mmol) of 60 % sodium hydride was added and the mixture was stirred at room temperature for ten minutes and 0.40 g (1.3 mmol) of 2-(2-methoxyimino-1-methylpropylideneaminooxymethyl)-α-methoxyiminobenzyl chloride was added and the mixture was stirred at 110 °C for 2 hours. When the reaction was completed, 100 ml of ether was added, and the mixture was washed with brine

twice. The ether layer was dried over anhydrous magnesium sulfate and concentrated under reduced pressure. The residue was purified by silica gel column chromatography with a mature of ethyl acetate and n-hexane to give 0.23 g (yield 51.1 %) of 1-[α-methoxyimino-2-(2-methoxyimino-1-methylpropylideneaminooxymethyl)benzyl]imidazole as a colorless oil.

$^1$H-NMR ($CDCl_3$) δ ppm: 1.90 (3H, s), 1.91 (3H, s), 3.93 (3H, s), 4.04 (3H, s), 5.10 (2H, s), 7.03 (1H, s), 7.12 (1H, s), 7.34-7.50 (4H, m), 8.00 (1H, s).

<u>EXAMPLE 6</u>

Synthesis of 3-[α-methoxyimino-2-(2-dimethylhydrazono-1-methylpropylideneaminooxymethyl)benzyl]imidazole

(1) Synthesis of 3-[α-methoxyimino-2-(1-methyl-2-oxopropylideneaminooxymethyl)benzyl]isoxazole

To 2.95 g (10 mmol) of 3-(2-bromomethyl-α-methoxyiminobenzyl)isoxazole, 20 ml of methyl ethyl ketone, 1.52 g (15 mmol) of 3-oxo-2-butanoneoxime and 2.76 g (20 mmol) of potassium carbonate was added and the mixture was refluxed for 5 hours with stirring. When the reaction was completed, the insoluble material was separated and concentrated under reduced pressure and the residue was purified by silica gel column chromatography with a mixture of ethyl acetate and n-hexane to give 2.60 g (yield 82.5 %) of 3-[α-methoxyimino-2-(1-methyl-2-oxopropylideneaminooxymethyl)benzyl]isoxazole as a colorless crystal.

mp 86 to 87 °C (a part of the above crystal was recrystallized from a mixture of ethyl acetate and n-hexane)
$^1$H-NMR ($CDCl_3$) δ ppm: 1.73 (3H, s), 2.25 (3H, s), 4.00 (3H, s), 5.19 (2H, s), 6.76 (1H, d, J=1.8), 7.24-7.51 (4H, m), 8.38 (1H, d, J=1.8).

(2) Synthesis of 3-[α-methoxyimino-2-(2-dimethylhydrazono-1-methylpropylideneaminooxymethyl)benzyl]isoxazole

To 3 ml of methanol, 0.32 g (1 mmol) of 3-[α-methoxyimino-2-(1-methyl-2-oxopropylideneaminooxymethyl)benzyl]isoxazole was dissolved and 0.23 nil (3 mmol) of N,N-dimethylhydrazine was added, and the mixture was refluxed for 8 hours with stirring. When the reaction was completed, the mixture was concentrated under reduced pressure and the residue was purified by silica gel column chromatography with a mixture of ethyl acetate and n-hexane to give 0.19 g (yield 53.2 %) of 3-[α-methoxyimino-2-(2-dimethylhydrazono-1-methylpropylideneaminooxymethyl)benzyl]isoxazole as a colorless oil.

$^1$H-NMR ($CDCl_3$) δ ppm: 1.87 (3H, s), 1.99 (3H, s), 2.54 (6H, s), 4.00 (3H, s), 5.09 (2H, s), 6.74 (1H, d, J=1.8), 7.24-7.53 (4H, m), 8.37 (1H, d, J=1.8).

<u>EXAMPLE 7</u>

Synthesis of 3-[2-(3-methoxyimino-2-butyloxyiminomethyl)-α-methoxyiminobenzyl]isoxazole

(1) Synthesis of 3-(2-formyl-α-methoxyiminobenzyl)isoxazole

To 65 ml of a dichloromethane solution containing 3.12 ml (36.4 mmol) of oxalyl chloride was added dropwise 2.77 ml (39 mmol) of DMSO at a temperature below - 60 °C over 4 minutes and the resultant mixture was stirred for 16 minutes at -70 to - 60 °C. And then 3.02 g (13 mmol) of 3-(2-hydroxymethyl-α-methoxyiminobenzyl)isoxazole in 26 ml of dichloromethane was added dropwise over 25 minutes at a temperature below -60 °C and the resultant mixture was stirred at - 70 to -60 °C for 25 minutes. To the reaction mixture was added dropwise 9.06 ml (65 mmol) of triethylamine at a temperature below -60 °C for 5 minutes and the mixture was stirred at -70 °C to -10 °C for 1.5 hours. After the reaction completion, 250 ml of a half saturated aqueous solution of ammonium chloride was added and the mixture was extracted with 150 ml of dichloromethane twice. The dichloromethane layer was dried over anhydrous magnesium sulfate and concentrated under reduced pressure. The residue was purified by silica gel column chromatography with a mixture of ethyl acetate and n-hexane to give 2.38 g (yield 78.9 %) of 3-(2-formyl-α-methoxyiminobenzyl)isoxazole as a colorless crystal.

$^1$H-NMR ($CDCl_3$) δ ppm 3.99 (3H, s). 6.87 (1H, d, J=1.8), 7.40 (1H, d, J=7.3), 7.61 (1H, t, J=7.9), 7.71 (1H, td, J=7.3, 2.3), 8.01 (1H, d, J=7.3), 8.43 (1H, d, J=1.8), 9.88 (1H, s).

(2) Synthesis of 3-(2-hydorxyiminomethyl-α-methoxyiminobenzyl)isoxazole

In a solution of 12 ml of methanol was dissolved 1.38 g (6 mmol) of 3-(2-formyl-α-methoxyiminobenzyl)isoxazole, and 0.83 g (12 mmol) of hydroxylamine hydrochloride and 1.31 ml (16.2 mmol) of pyridine was added and the mixture was refluxed for 3 hours with stirring. When the reaction was completed, 150 ml of 0.1 N hydrochloric acid solution was added and 100 ml of dichloromethane twice. The dichloromethane layer was dried over anhydrous magnesium sulfate and concentrated under reduced pressure. The residue was purified by silica gel column chromatography with a mixture of ethyl acetate and n-hexane to give 1.04 g (yield 70.7 %) of 3-(2-hydorxyiminomethyl-α-methoxyiminobenzyl)isoxazole as a colorless crystal.

$^1$H-NMR ($CDCl_3$) δ ppm 4.00 (3H, s), 6.79 (1H, d, J=1.8), 7.24-7.51 (4H, m), 7.87-7.91 (1H, m), 7.97 (1H, s), 8.41 (1H, d, J=1.8).

(3) Synthesis of 3-[2-(3-oxo-2-butyloxyiminomethyl)-α-methoxyiminobenzyl]isoxazole

To 0.37 g (1.5 mmol) of 3-(2-hydoroxyiminomethyl-α-methoxyiminobenzyl)isoxazole, 3 ml of DMF, 0.30 ml (3 mmol) of 3-chloro-2-butanone and 0.09 g (2.25 mmol) of 60 % sodium hydride was added under ice cooling and the mixture was stirred at room temperature for 4 hours. To the reaction mixture, 100 ml of ether was added and washed with 80 ml of brine twice. The ether layer was dried over anhydrous magnesium sulfate and concentrated under reduced pressure. The residue was purified by silica gel column chromatography with a mixture of ethyl acetate and n-hexane to give 0.30 g (yield 63.4 %) of 3-[2-(3-oxo-2-butyloxyiminomethyl)-α-methoxyiminobenzyl]isoxazole as a colorless oil.

$^1$H-NMR ($CDCl_3$) δ ppm: 1.35 (3H, d, J=6.7), 2.12 (3H, s), 3.99 (3H, s), 4.54 (1H, q, J=1.8), 6.78 (1H, d, J=1.8), 7.23-7.30 (1H, m), 7.40-7.51 (2H, m), 7.79-7.82 (1H, m), 8.02 (1H, s), 8.40 (1H, d, J=1.8).

(4) Synthesis of 3-[2-(3-methoxyimino-2-butyloxyiminomethyl)-α-methoxyiminobenzyl]isoxazole

In a solution of 2 ml of methanol was dissolved 0.19 g (0.6 mmol) of 3-[2-(3-oxo-2-butyloxyiminomethyl)-α-methoxyiminobenzyl]isoxazole, 0.08 g (0.9 mmol) of methoxylamine hydrochloride and 0.08 g (1.02 mmol) of pyridine was added and the mixture was refluxed for 1 hour with stirring. When the reaction was completed, 100 ml of 0.1 N hydrochloric acid solution was added and the mixture was extracted with 50 ml of dichloromethane twice. The dichloromethane layer was dried over anhydrous magnesium sulfate and concentrated under reduced pressure. The residue was purified by silica gel column chromatography with a mixture of ethyl acetate and n-hexane to give 0.18 g (yield 87.1 %) of 3-[2-(3-methoxyimino-2-butyloxyiminomethyl)-α-methoxyiminobenzyl]isoxazole as a colorless oil.

$^1$H-NMR ($CDCl_3$) δ ppm: 1.36 (3H, d, J=6.7), 1.76 (3H, s), 3.89 (3H, s), 3.98 (3H, s), 4.74 (1H, q, J=6.7), 6.79 (1H, d, J=1.2), 7.24-7.26 (1H, m), 7.40-7.48 (2H, m), 7.84-7.90 (1H, m), 7.93 (1H, a), 8.39 (1H, d, J=1.2).

EXAMPLE 8

Synthesis of 3-[2-(2-isopropoxyimino-1-methylpropylideneaminooxymethyl)-α-methoxyiminobenzyl]isoxazole

(1) Synthesis of 3-[2-(2-hydroxyimino-1-methylpropylideneaminooxymethyl)-α-methoxyiminobenzyl]isoxazole

In a solution of 10 ml of methanol was dissolved 1.58 g (5 mmol) of 3-[α-methoxyimino-2-(1-methyl-2-oxopropylideneaminooxymethyl)benzyl]isoxazole, 0.52 g (7.5 mmol) of hydoxylamine hydorochloride and 0.67 g (8.5 mmol) of pyridine was added and the mixture was refluxed for a hour with stirring. After the reaction completion, 150 ml of 0.1 N hydrochloric acid solution was added and the mixture was extracted with 100 ml of dichloromethane twice. The dichloromethane layer was dried over anhydrous magnesium sulfate and concentrated under reduced pressure. The residue was recrystallized from a mixture of ethyl acetate and n-hexane to give 1.26 g (yield 76.3 %) of 3-[2-(2-hydroxyimino-1-methylpropylideneaminooxymethyl)-α-methoxyiminobenzyl]isoxazole as a colorless crystal.

mp 109 to 110 °C
$^1$H-NMR ($CDCl_3$) δ ppm: 1.83 (3H, s), 1.96 (3H, s), 4.00 (3H, s), 5.10 (2H, s), 6.74 (1H, d, J=1.8), 7.24-7.27 (1H, m), 7.37-7.52 (4H, m), 8.38 (1H, d, J=1.8).

(2) Synthesisof 3-[2-(2-isopropoxyimino-1-methylpropylideneaminooxymethyl)-α-methoxyiminobenzyl]isoxazole

To 0.33 g (1 mmol) of 3-[2-(2-hydoroxyimino-1-methylpropylideneaminooxymethyl)-α-methoxyiminobenzyl]isoxazole, 2 ml of DMF, 0.13 ml (1.3 mmol) of isopropyl iodide and 0.05 g (1.3 mmol) of 60 % sodium hydride was added under ice cooling and the mixture was stirred at room temperature for 2 hours. To the reaction mixture, 100 ml of ether was added and the mixture was washed with 80 ml of brine twice. The ether layer was dried over anhydrous magnesium sulfate and concentrated under reduced pressure. The residue was purified by silica gel column chromatography with a mixture of ethyl acetate and n-hexane, and was recrystallized from n-hexane to give 0.30 g (yield 63.4 %) of 3-[2-(2-isopropoxyimino-1-methylpropylideneaminooxymethyl)-α-methoxyiminobenzyl]isoxazole as a colorless crystal.

mp 97 to 98 °C
$^1$H-NMR ($CDCl_3$) δ ppm: 1.24 (6H, d, J=6.1), 1.86 (3H, s), 1.90 (3H, s), 4.00 (3H, s), 4.35 (1H, sept, J=6.1), 5.08 (2H, s), 6.74 (1H, d, J=1.8), 7.23-7.52 (4H, m), 8.37 (1H, d, J=1.2).

EXAMPLE 9

Synthesis of 2-[α-methoxyimino-2-(2-methoxyimino-1-methylpropylideneaminooxymethyl)benzyl]-1,3,4-oxadiazole

(1) Synthesis of α-methoxyimino-2-(2-methoxyimino-1-methylpropylideneaminooxymethyl)phenylacetic hydrazido

To 1.01 g (3 mmol) of methyl α-methoxyimino-2-(2-methoxyimino-1-methylpropylideneaminooxymethyl)phenylacetate, 6 ml of methanol, 6 ml of THF and 0.6 g (12 mmol) of hydrazine mono hydrate was added and the mixture was stirred at room temperature for 2 days. After the reaction completion, 200 ml of water was added and mixture was extracted with 80 ml of dichloromethane twice. The dichloromethane layer was dried over anhydrous magnesium sulfate and concentrated under reduced pressure. The residue was recrystallized from a mixture of ethyl acetate and n-hexane to give 0.80 g (yield 79.2 %) of α-methoxyimino-2-(2-methoxyimino-1-methylpropylideneaminooxymethyl)phenylacetic hydrazido as a colorless oil.

$^1$H-NMR ($CDCl_3$) δ ppm: 1.95 (3H, s), 1.99 (3H, s), 3.91 (2H, brs), 3.93 (3H, s), 3.96 (3H, s), 5.06 (2H, s), 7.17-7.20 (1H, m), 7.34-7.48 (3H, m), 7.77 (1H, s).

(2) Synthesis of 2-[α-methoxyimino-2-(2-methoxyimino-1-methylpropylideneaminooxymethyl)benzyl]-1,3,4-oxadiazole

To 0.34 g (1 mmol) of α-methoxyimino-2-(2-methoxyimino-1-methylpropylideneaminooxymethyl)phenylacetic hydrazido, 1 ml of ethyl orthoformate was added and the mixture was refluxed for 1 hour with stirring, and concentrated under reduced pressure. To the residue was 2 ml of benzene and 0.02 g (0.1 mmol) of p-toluenesulfonic acid mono hydrate was added and the mixture was refluxed for 45 minutes with stirring. To the reaction mixture, 100 ml of ether was added and the mixture was washed with 80 ml of brine twice. The ether layer was dried over anhydrous magnesium sulfate and concentrated under reduced pressure. The residue was purified by silica gel column chromatography with a mixture of ethyl acetate and n-hexane and recrystallized from a mixture of ethyl acetate and n-hexane to give 0.23 g (yield 65.7%) of 2-[α-methoxyimino-2-(2-methoxyimino-1-methylpropylideneaminooxymethyl)benzyl]-1,3,4-oxadiazole as a colorless crystal.

mp 110 to 111 °C
$^1$H-NMR ($CDCl_3$) δ ppm: 1.84 (3H, s), 1.89 (3H, s), 3.92 (3H, s), 4.09 (3H, s), 5.10 (2H, s), 7.29-7.32 (1H, m), 7.40-7.53 (3H, m), 8.43 (1H, s).

The compounds represented by the formula (I) which can be produced in the same manner as in the above-mentioned EXAMPLE are exemplified by a series of the compounds 1 to 14 as shown below and the combination of the substituents consiting of A, B, $R^4$ and $R^5$ in the compounds 1 to 14 are shown in Tables 1 to 22. The physical constants of their compounds are indicated in the Tables 23 and 24. The physical constants of the compounds produced in EXAMPLE are also shown in the Tables. The compound numbers in the Tables 23 and 24 and in the following Test Examples consist of the series number of the compounds and the combination number of the substituents, and, for example, the compound number 3-2 means that the series number of the compounds is 3 and the combination number of the substituents is 2.

A series of the compounds

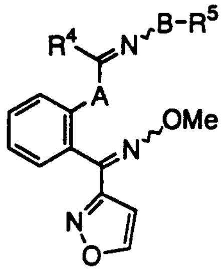

1

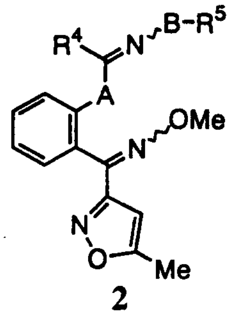

2

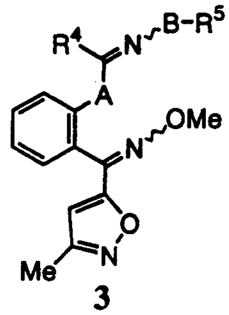

3

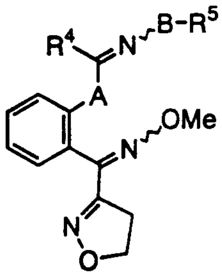

4

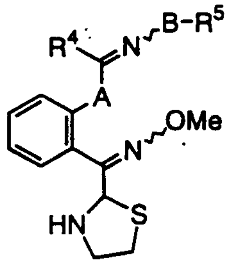

5

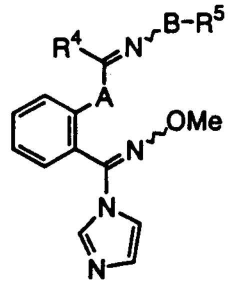

6

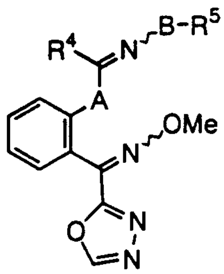

7

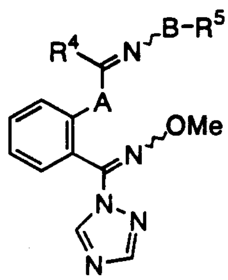

8

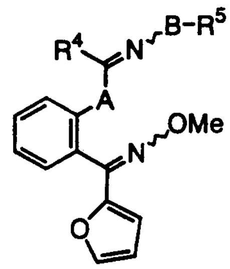

9

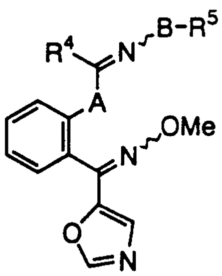

10

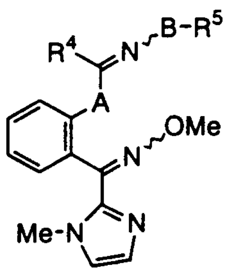

11

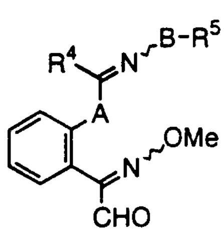

12

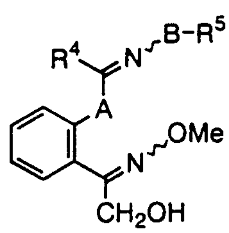

13

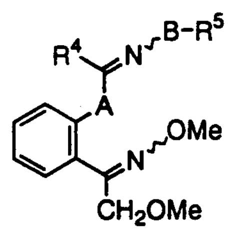

14

Table 1

| No. | A | B | $R^4$ | $R^5$ |
|---|---|---|---|---|
| 1 | $-CH_2ON=C(Me)-$ | O | Me | H |
| 2 | $-CH_2ON=C(Me)-$ | O | Me | Me |
| 3 | $-CH_2ON=C(Me)-$ | O | Me | Et |
| 4 | $-CH_2ON=C(Me)-$ | O | Me | n-Pr |
| 5 | $-CH_2ON=C(Me)-$ | O | Me | i-Pr |
| 6 | $-CH_2ON=C(Me)-$ | O | Me | n-Bu |
| 7 | $-CH_2ON=C(Me)-$ | O | Me | i-Bu |
| 8 | $-CH_2ON=C(Me)-$ | O | Me | s-Bu |
| 9 | $-CH_2ON=C(Me)-$ | O | Me | n-Pentyl |
| 10 | $-CH_2ON=C(Me)-$ | O | Me | n-Hexyl |
| 11 | $-CH_2ON=C(Me)-$ | O | Me | $CHF_2$ |
| 12 | $-CH_2ON=C(Me)-$ | O | Me | Allyl |
| 13 | $-CH_2ON=C(Me)-$ | O | Me | 2-Propynyl |
| 14 | $-CH_2ON=C(Me)-$ | O | Me | 2-Butenyl |
| 15 | $-CH_2ON=C(Me)-$ | O | Me | 2-Oxopropyl |
| 16 | $-CH_2ON=C(Me)-$ | O | Me | MeOC(=O)- |
| 17 | $-CH_2ON=C(Me)-$ | O | Me | EtOC(=O)- |
| 18 | $-CH_2ON=C(Me)-$ | O | Me | $EtOC(=O)CH_2-$ |
| 19 | $-CH_2ON=C(Me)-$ | O | Me | EtOC(=O)CH(Me)- |
| 20 | $-CH_2ON=C(Me)-$ | O | Me | $MeOC(=O)CH_2-$ |

Table 2

| No. | A | B | $R^4$ | $R^5$ |
|---|---|---|---|---|
| 21 | $-CH_2ON=C(Me)-$ | O | Me | Ph |
| 22 | $-CH_2ON=C(Me)-$ | O | Me | Benzyl |
| 23 | $-CH_2ON=C(Me)-$ | O | Me | α-Me-Benzyl |
| 24 | $-CH_2ON=C(Me)-$ | O | Me | 2-Me-Benzyl |
| 25 | $-CH_2ON=C(Me)-$ | O | Me | 3-Me-Benzyl |
| 26 | $-CH_2ON=C(Me)-$ | O | Me | 4-Me-Benzyl |
| 27 | $-CH_2ON=C(Me)-$ | O | Me | 2-Cl-Benzyl |
| 28 | $-CH_2ON=C(Me)-$ | O | Me | 3-Cl-Benzyl |
| 29 | $-CH_2ON=C(Me)-$ | O | Me | 4-Cl-Benzyl |
| 30 | $-CH_2ON=C(Me)-$ | O | Me | $MeOCH_2-$ |
| 31 | $-CH_2ON=C(Me)-$ | O | Me | $EtOCH_2-$ |
| 32 | $-CH_2ON=C(Me)-$ | O | Me | $MeO(CH_2)_2-$ |
| 33 | $-CH_2ON=C(Me)-$ | O | Me | $EtO(CH_2)_2-$ |
| 34 | $-CH_2ON=C(Me)-$ | O | Me | Acetyl |
| 35 | $-CH_2ON=C(Me)-$ | O | Me | $NCCH_2-$ |
| 36 | $-CH_2ON=C(Me)-$ | O | Me | $NC(CH_2)_2-$ |
| 37 | $-CH_2ON=C(Me)-$ | O | Me | c-Pentyl |
| 38 | $-CH_2ON=C(Me)-$ | O | Me | c-Pentyl$CH_2-$ |
| 39 | $-CH_2ON=C(Me)-$ | O | Me | 5-Me-isoxazol -3-ylmethyl |
| 40 | $-CH_2ON=C(Me)-$ | O | Me | $CF_3CH_2-$ |

Table 3

| No. | A | B | $R^4$ | $R^5$ |
|---|---|---|---|---|
| 41 | $-CH_2ON=C(Me)-$ | O | Et | H |
| 42 | $-CH_2ON=C(Me)-$ | O | Et | Me |
| 43 | $-CH_2ON=C(Me)-$ | O | Et | Et |
| 44 | $-CH_2ON=C(Me)-$ | O | Et | n-Pr |
| 45 | $-CH_2ON=C(Me)-$ | O | Et | i-Pr |
| 46 | $-CH_2ON=C(Me)-$ | O | Et | n-Bu |
| 47 | $-CH_2ON=C(Me)-$ | O | Et | i-Bu |
| 48 | $-CH_2ON=C(Me)-$ | O | Et | s-Bu |
| 49 | $-CH_2ON=C(Me)-$ | O | Et | n-Pentyl |
| 50 | $-CH_2ON=C(Me)-$ | O | Et | n-Hexyl |
| 51 | $-CH_2ON=C(Me)-$ | O | Et | $CHF_2$ |
| 52 | $-CH_2ON=C(Me)-$ | O | Et | Allyl |
| 53 | $-CH_2ON=C(Me)-$ | O | Et | 2-Propynyl |
| 54 | $-CH_2ON=C(Me)-$ | O | Et | 2-Butenyl |
| 55 | $-CH_2ON=C(Me)-$ | O | Et | 2-Oxopropyl |
| 56 | $-CH_2ON=C(Me)-$ | O | Et | MeOC(=O)- |
| 57 | $-CH_2ON=C(Me)-$ | O | Et | EtOC(=O)- |
| 58 | $-CH_2ON=C(Me)-$ | O | Et | $EtOC(=O)CH_2-$ |
| 59 | $-CH_2ON=C(Me)-$ | O | Et | EtOC(=O)CH(Me)- |
| 60 | $-CH_2ON=C(Me)-$ | O | Et | $MeOC(=O)CH_2-$ |

Table 4

| No. | A | B | $R^4$ | $R^5$ |
|---|---|---|---|---|
| 61 | $-CH_2ON=C(Me)-$ | O | Et | Ph |
| 62 | $-CH_2ON=C(Me)-$ | O | Et | Benzyl |
| 63 | $-CH_2ON=C(Me)-$ | O | Et | α-Me-Benzyl |
| 64 | $-CH_2ON=C(Me)-$ | O | Et | 2-Me-Benzyl |
| 65 | $-CH_2ON=C(Me)-$ | O | Et | 3-Me-Benzyl |
| 66 | $-CH_2ON=C(Me)-$ | O | Et | 4-Me-Benzyl |
| 67 | $-CH_2ON=C(Me)-$ | O | Et | 2-Cl-Benzyl |
| 68 | $-CH_2ON=C(Me)-$ | O | Et | 3-Cl-Benzyl |
| 69 | $-CH_2ON=C(Me)-$ | O | Et | 4-Cl-Benzyl |
| 70 | $-CH_2ON=C(Me)-$ | O | Et | $MeOCH_2-$ |
| 71 | $-CH_2ON=C(Me)-$ | O | Et | $EtOCH_2-$ |
| 72 | $-CH_2ON=C(Me)-$ | O | Et | $MeO(CH_2)_2-$ |
| 73 | $-CH_2ON=C(Me)-$ | O | Et | $EtO(CH_2)_2-$ |
| 74 | $-CH_2ON=C(Me)-$ | O | Et | Acetyl |
| 75 | $-CH_2ON=C(Me)-$ | O | Et | $NCCH_2-$ |
| 76 | $-CH_2ON=C(Me)-$ | O | Et | $NC(CH_2)_2-$ |
| 77 | $-CH_2ON=C(Me)-$ | O | Et | c-Pentyl |
| 78 | $-CH_2ON=C(Me)-$ | O | Et | c-Pentyl$CH_2-$ |
| 79 | $-CH_2ON=C(Me)-$ | O | Et | 5-Me-isoxazol -3-ylmethyl |
| 80 | $-CH_2ON=C(Me)-$ | O | Et | $CF_3CH_2-$ |

Table 5

| No. | A | B | $R^4$ | $R^5$ |
|---|---|---|---|---|
| 81 | $-CH_2ON=C(Et)-$ | O | Me | H |
| 82 | $-CH_2ON=C(Et)-$ | O | Me | Me |
| 83 | $-CH_2ON=C(Et)-$ | O | Me | Et |
| 84 | $-CH_2ON=C(Et)-$ | O | Me | n-Pr |
| 85 | $-CH_2ON=C(Et)-$ | O | Me | i-Pr |
| 86 | $-CH_2ON=C(Et)-$ | O | Me | n-Bu |
| 87 | $-CH_2ON=C(Et)-$ | O | Me | i-Bu |
| 88 | $-CH_2ON=C(Et)-$ | O | Me | s-Bu |
| 89 | $-CH_2ON=C(Et)-$ | O | Me | n-Pentyl |
| 90 | $-CH_2ON=C(Et)-$ | O | Me | n-Hexyl |
| 91 | $-CH_2ON=C(Et)-$ | O | Me | $CHF_2$ |
| 92 | $-CH_2ON=C(Et)-$ | O | Me | Allyl |
| 93 | $-CH_2ON=C(Et)-$ | O | Me | 2-Propynyl |
| 94 | $-CH_2ON=C(Et)-$ | O | Me | 2-Butenyl |
| 95 | $-CH_2ON=C(Et)-$ | O | Me | 2-Oxopropyl |
| 96 | $-CH_2ON=C(Et)-$ | O | Me | MeOC(=O)- |
| 97 | $-CH_2ON=C(Et)-$ | O | Me | EtOC(=O)- |
| 98 | $-CH_2ON=C(Et)-$ | O | Me | $EtOC(=O)CH_2-$ |
| 99 | $-CH_2ON=C(Et)-$ | O | Me | EtOC(=O)CH(Me)- |
| 100 | $-CH_2ON=C(Et)-$ | O | Me | $MeOC(=O)CH_2-$ |

Table 6

| No. | A | B | $R^4$ | $R^5$ |
|---|---|---|---|---|
| 101 | $-CH_2ON=C(Et)-$ | Me | Ph | |
| 102 | $-CH_2ON=C(Et)-$ | O | Me | Benzyl |
| 103 | $-CH_2ON=C(Et)-$ | O | Me | α-Me-Benzyl |
| 104 | $-CH_2ON=C(Et)-$ | O | Me | 2-Me-Benzyl |
| 105 | $-CH_2ON=C(Et)-$ | O | Me | 3-Me-Benzyl |
| 106 | $-CH_2ON=C(Et)-$ | O | Me | 4-Me-Benzyl |
| 107 | $-CH_2ON=C(Et)-$ | O | Me | 2-Cl-Benzyl |
| 108 | $-CH_2ON=C(Et)-$ | O | Me | 3-Cl-Benzyl |
| 109 | $-CH_2ON=C(Et)-$ | O | Me | 4-Cl-Benzyl |
| 110 | $-CH_2ON=C(Et)-$ | O | Me | $MeOCH_2-$ |
| 111 | $-CH_2ON=C(Et)-$ | O | Me | $EtOCH_2-$ |
| 112 | $-CH_2ON=C(Et)-$ | O | Me | $MeO(CH_2)_2-$ |
| 113 | $-CH_2ON=C(Et)-$ | O | Me | $EtO(CH_2)_2-$ |
| 114 | $-CH_2ON=C(Et)-$ | O | Me | Acetyl |
| 115 | $-CH_2ON=C(Et)-$ | O | Me | $NCCH_2-$ |
| 116 | $-CH_2ON=C(Et)-$ | O | Me | $NC(CH_2)_2-$ |
| 117 | $-CH_2ON=C(Et)-$ | O | Me | c-Pentyl |
| 118 | $-CH_2ON=C(Et)-$ | O | Me | c-Pentyl$CH_2-$ |
| 119 | $-CH_2ON=C(Et)-$ | O | Me | 5-Me-isoxazol -3-ylmethyl |
| 120 | $-CH_2ON=C(Et)-$ | O | Me | $CF_3CH_2-$ |

Table 7

| No. | A | B | $R^4$ | $R^5$ |
|---|---|---|---|---|
| 121 | $-CH_2ON=C(Et)-$ | O | Et | H |
| 122 | $-CH_2ON=C(Et)-$ | O | Et | Me |
| 123 | $-CH_2ON=C(Et)-$ | O | Et | Et |
| 124 | $-CH_2ON=C(Et)-$ | O | Et | n-Pr |
| 125 | $-CH_2ON=C(Et)-$ | O | Et | i-Pr |
| 126 | $-CH_2ON=C(Et)-$ | O | Et | n-Bu |
| 127 | $-CH_2ON=C(Et)-$ | O | Et | i-Bu |
| 128 | $-CH_2ON=C(Et)-$ | O | Et | s-Bu |
| 129 | $-CH_2ON=C(Et)-$ | O | Et | n-Pentyl |
| 130 | $-CH_2ON=C(Et)-$ | O | Et | n-Hexyl |
| 131 | $-CH_2ON=C(Et)-$ | O | Et | $CHF_2$ |
| 132 | $-CH_2ON=C(Et)-$ | O | Et | Allyl |
| 133 | $-CH_2ON=C(Et)-$ | O | Et | 2-Propynyl |
| 134 | $-CH_2ON=C(Et)-$ | O | Et | 2-Butenyl |
| 135 | $-CH_2ON=C(Et)-$ | O | Et | 2-Oxopropyl |
| 136 | $-CH_2ON=C(Et)-$ | O | Et | MeOC(=O)- |
| 137 | $-CH_2ON=C(Et)-$ | O | Et | EtOC(=O)- |
| 138 | $-CH_2ON=C(Et)-$ | O | Et | $EtOC(=O)CH_2-$ |
| 139 | $-CH_2ON=C(Et)-$ | O | Et | EtOC(=O)CH(Me)- |
| 140 | $-CH_2ON=C(Et)-$ | O | Et | $MeOC(=O)CH_2-$ |

Table 8

| No. | A | B | $R^4$ | $R^5$ |
|---|---|---|---|---|
| 141 | $-CH_2ON=C(Et)-$ | O | Et | Ph |
| 142 | $-CH_2ON=C(Et)-$ | O | Et | Benzyl |
| 143 | $-CH_2ON=C(Et)-$ | O | Et | α-Me-Benzyl |
| 144 | $-CH_2ON=C(Et)-$ | O | Et | 2-Me-Benzyl |
| 145 | $-CH_2ON=C(Et)-$ | O | Et | 3-Me-Benzyl |
| 146 | $-CH_2ON=C(Et)-$ | O | Et | 4-Me-Benzyl |
| 147 | $-CH_2ON=C(Et)-$ | O | Et | 2-Cl-Benzyl |
| 148 | $-CH_2ON=C(Et)-$ | O | Et | 3-Cl-Benzyl |
| 149 | $-CH_2ON=C(Et)-$ | O | Et | 4-Cl-Benzyl |
| 150 | $-CH_2ON=C(Et)-$ | O | Et | $MeOCH_2-$ |
| 151 | $-CH_2ON=C(Et)-$ | O | Et | $EtOCH_2-$ |
| 152 | $-CH_2ON=C(Et)-$ | O | Et | $MeO(CH_2)_2-$ |
| 153 | $-CH_2ON=C(Et)-$ | O | Et | $EtO(CH_2)_2-$ |
| 154 | $-CH_2ON=C(Et)-$ | O | Et | Acetyl |
| 155 | $-CH_2ON=C(Et)-$ | O | Et | $NCCH_2-$ |
| 156 | $-CH_2ON=C(Et)-$ | O | Et | $NC(CH_2)_2-$ |
| 157 | $-CH_2ON=C(Et)-$ | O | Et | c-Pentyl |
| 158 | $-CH_2ON=C(Et)-$ | O | Et | c-Pentyl$CH_2-$ |
| 159 | $-CH_2ON=C(Et)-$ | O | Et | 5-Me-isoxazol -3-ylmethyl |
| 160 | $-CH_2ON=C(Et)-$ | O | Et | $CF_3CH_2-$ |

Table 9

| No. | A | B | $R^4$ | $R^5$ |
|---|---|---|---|---|
| 161 | -CH=NOCH(Me)- | O | Me | H |
| 162 | -CH=NOCH(Me)- | O | Me | Me |
| 163 | -CH=NOCH(Me)- | O | Me | Et |
| 164 | -CH=NOCH(Me)- | O | Me | n-Pr |
| 165 | -CH=NOCH(Me)- | O | Me | i-Pr |
| 166 | -CH=NOCH(Me)- | O | Me | n-Bu |
| 167 | -CH=NOCH(Me)- | O | Me | i-Bu |
| 168 | -CH=NOCH(Me)- | O | Me | s-Bu |
| 169 | -CH=NOCH(Me)- | O | Me | n-Pentyl |
| 170 | -CH=NOCH(Me)- | O | Me | n-Hexyl |
| 171 | -CH=NOCH(Me)- | O | Me | $CHF_2$ |
| 172 | -CH=NOCH(Me)- | O | Me | Allyl |
| 173 | -CH=NOCH(Me)- | O | Me | 2-Propynyl |
| 174 | -CH=NOCH(Me)- | O | Me | 2-Butenyl |
| 175 | -CH=NOCH(Me)- | O | Me | 2-Oxopropyl |
| 176 | -CH=NOCH(Me)- | O | Me | MeOC(=O)- |
| 177 | -CH=NOCH(Me)- | O | Me | EtOC(=O)- |
| 178 | -CH=NOCH(Me)- | O | Me | EtOC(=O)$CH_2$- |
| 179 | -CH=NOCH(Me)- | O | Me | EtOC(=O)CH(Me)- |
| 180 | -CH=NOCH(Me)- | O | Me | MeOC(=O)$CH_2$- |

Table 10

| No. | A | B | $R^4$ | $R^5$ |
|---|---|---|---|---|
| 181 | -CH=NOCH(Me)- | O | Me | Ph |
| 182 | -CH=NOCH(Me)- | O | Me | Benzyl |
| 183 | -CH=NOCH(Me)- | O | Me | α-Me-Benzyl |
| 184 | -CH=NOCH(Me)- | O | Me | 2-Me-Benzyl |
| 185 | -CH=NOCH(Me)- | O | Me | 3-Me-Benzyl |
| 186 | -CH=NOCH(Me)- | O | Me | 4-Me-Benzyl |
| 187 | -CH=NOCH(Me)- | O | Me | 2-Cl-Benzyl |
| 188 | -CH=NOCH(Me)- | O | Me | 3-Cl-Benzyl |
| 189 | -CH=NOCH(Me)- | O | Me | 4-Cl-Benzyl |
| 190 | -CH=NOCH(Me)- | O | Me | $MeOCH_2$- |
| 191 | -CH=NOCH(Me)- | O | Me | $EtOCH_2$- |
| 192 | -CH=NOCH(Me)- | O | Me | $MeO(CH_2)_2$- |
| 193 | -CH=NOCH(Me)- | O | Me | $EtO(CH_2)_2$- |
| 194 | -CH=NOCH(Me)- | O | Me | Acetyl |
| 195 | -CH=NOCH(Me)- | O | Me | $NCCH_2$- |
| 196 | -CH=NOCH(Me)- | O | Me | $NC(CH_2)_2$- |
| 197 | -$CH_2$ON=C(Me)- | O | Me | c-Pentyl |
| 198 | -CH=NOCH(Me)- | O | Me | c-Pentyl$CH_2$- |
| 199 | -CH=NOCH(Me)- | O | Me | 5-Me-isoxazol -3-ylmethyl |
| 200 | -CH=NOCH(Me)- | O | Me | $CF_3CH_2$- |

Table 11

| No. | A | B | $R^4$ | $R^5$ |
|---|---|---|---|---|
| 201 | $-CH_2ON=C(CN)-$ | O | Me | Me |
| 202 | $-CH_2ON=C(CN)-$ | O | Me | Et |
| 203 | $-CH_2ON=C(CN)-$ | O | Me | n-Pr |
| 204 | $-CH_2ON=C(CN)-$ | O | Me | i-Pr |
| 205 | $-CH_2ON=C(CN)-$ | O | Me | Allyl |
| 206 | $-CH_2ON=C(CN)-$ | O | Me | 2-Propynyl |
| 207 | $-CH_2ON=C(CN)-$ | O | Me | Benzyl |
| 208 | $-CH_2ON=C(CN)-$ | O | Me | 4-Cl-Benzyl |
| 209 | $-CH_2ON=C(CN)-$ | O | Me | $MeOCH_2-$ |
| 210 | $-CH_2ON=C(CN)-$ | O | Me | $EtOCH_2-$ |
| 211 | $-CH_2ON=C(CN)-$ | O | Et | Me |
| 212 | $-CH_2ON=C(CN)-$ | O | Et | Et |
| 213 | $-CH_2ON=C(CN)-$ | O | Et | n-Pr |
| 214 | $-CH_2ON=C(CN)-$ | O | Et | i-Pr |
| 215 | $-CH_2ON=C(CN)-$ | O | Et | Allyl |
| 216 | $-CH_2ON=C(CN)-$ | O | Et | 2-Propynyl |
| 217 | $-CH_2ON=C(CN)-$ | O | Et | Benzyl |
| 218 | $-CH_2ON=C(CN)-$ | O | Et | 4-Cl-Benzyl |
| 219 | $-CH_2ON=C(CN)-$ | O | Et | $MeOCH_2-$ |
| 220 | $-CH_2ON=C(CN)-$ | O | Et | $EtOCH_2-$ |

Table 12

| No. | A | B | $R^4$ | $R^5$ |
|---|---|---|---|---|
| 221 | $-CH_2ON=C(Me)-$ | O | Ph | Me |
| 222 | $-CH_2ON=C(Me)-$ | O | Ph | Et |
| 223 | $-CH_2ON=C(Me)-$ | O | Ph | n-Pr |
| 224 | $-CH_2ON=C(Me)-$ | O | Ph | i-Pr |
| 225 | $-CH_2ON=C(Me)-$ | O | Ph | Allyl |
| 226 | $-CH_2ON=C(Me)-$ | O | Ph | 2-Propynyl |
| 227 | $-CH_2ON=C(Me)-$ | O | Ph | Benzyl |
| 228 | $-CH_2ON=C(Me)-$ | O | Ph | 4-Cl-Benzyl |
| 229 | $-CH_2ON=C(Me)-$ | O | Ph | $MeOCH_2-$ |
| 230 | $-CH_2ON=C(Me)-$ | O | Ph | $EtOCH_2-$ |
| 231 | $-CH_2ON=C(Me)-$ | O | COOMe | Me |
| 232 | $-CH_2ON=C(Me)-$ | O | COOMe | Et |
| 233 | $-CH_2ON=C(Me)-$ | O | COOMe | n-Pr |
| 234 | $-CH_2ON=C(Me)-$ | O | COOMe | i-Pr |
| 235 | $-CH_2ON=C(Me)-$ | O | COOMe | Allyl |
| 236 | $-CH_2ON=C(Me)-$ | O | COOMe | 2-Propynyl |
| 237 | $-CH_2ON=C(Me)-$ | O | COOMe | Benzyl |
| 238 | $-CH_2ON=C(Me)-$ | O | COOMe | 4-Cl-Benzyl |
| 239 | $-CH_2ON=C(Me)-$ | O | COOMe | $MeOCH_2-$ |
| 240 | $-CH_2ON=C(Me)-$ | O | COOMe | $EtOCH_2-$ |

Table 13

| No. | A | B | $R^4$ | $R^5$ |
|---|---|---|---|---|
| 241 | $-CH=NN=C(Me)-$ | O | Me | Me |
| 242 | $-CH=NN=C(Me)-$ | O | Me | Et |
| 243 | $-CH=NN=C(Me)-$ | O | Me | n-Pr |
| 244 | $-CH=NN=C(Me)-$ | O | Me | i-Pr |
| 245 | $-CH=NN=C(Me)-$ | O | Me | Allyl |
| 246 | $-CH=NN=C(Me)-$ | O | Me | 2-Propynyl |
| 247 | $-CH=NN=C(Me)-$ | O | Me | Benzyl |
| 248 | $-CH=NN=C(Me)-$ | O | Me | 4-Cl-Benzyl |
| 249 | $-CH=NN=C(Me)-$ | O | Me | $MeOCH_2-$ |
| 250 | $-CH=NN=C(Me)-$ | O | Me | $EtOCH_2-$ |
| 251 | $-CH_2N(Ac)N=C(Me)-$ | O | Me | Me |
| 252 | $-CH_2N(Ac)N=C(Me)-$ | O | Me | Et |
| 253 | $-CH_2N(Ac)N=C(Me)-$ | O | Me | n-Pr |
| 254 | $-CH_2N(Ac)N=C(Me)-$ | O | Me | i-Pr |
| 255 | $-CH_2N(Ac)N=C(Me)-$ | O | Me | Allyl |
| 256 | $-CH_2N(Ac)N=C(Me)-$ | O | Me | 2-Propynyl |
| 257 | $-CH_2N(Ac)N=C(Me)-$ | O | Me | Benzyl |
| 258 | $-CH_2N(Ac)N=C(Me)-$ | O | Me | 4-Cl-Benzyl |
| 259 | $-CH_2N(Ac)N=C(Me)-$ | O | Me | $MeOCH_2-$ |
| 260 | $-CH_2N(Ac)N=C(Me)-$ | O | Me | $EtOCH_2-$ |

Table 14

| No. | A | B | $R^4$ | $R^5$ |
|---|---|---|---|---|
| 261 | $-CH_2ON=C(Me)-$ | NH | Me | Me |
| 262 | $-CH_2ON=C(Me)-$ | NH | Me | Et |
| 263 | $-CH_2ON=C(Me)-$ | NH | Me | n-Pr |
| 264 | $-CH_2ON=C(Me)-$ | NH | Me | i-Pr |
| 265 | $-CH_2ON=C(Me)-$ | NH | Me | Allyl |
| 266 | $-CH_2ON=C(Me)-$ | NH | Me | 2-Propynyl |
| 267 | $-CH_2ON=C(Me)-$ | NH | Me | Benzyl |
| 268 | $-CH_2ON=C(Me)-$ | NH | Me | 4-Cl-Benzyl |
| 269 | $-CH_2ON=C(Me)-$ | NH | Me | $MeOCH_2-$ |
| 270 | $-CH_2ON=C(Me)-$ | NH | Me | $EtOCH_2-$ |
| 271 | $-CH_2ON=C(Me)-$ | NMe | Me | Me |
| 272 | $-CH_2ON=C(Me)-$ | NMe | Me | Et |
| 273 | $-CH_2ON=C(Me)-$ | NMe | Me | n-Pr |
| 274 | $-CH_2ON=C(Me)-$ | NMe | Me | i-Pr |
| 275 | $-CH_2ON=C(Me)-$ | NMe | Me | Allyl |
| 276 | $-CH_2ON=C(Me)-$ | NMe | Me | 2-Propynyl |
| 277 | $-CH_2ON=C(Me)-$ | NMe | Me | Benzyl |
| 278 | $-CH_2ON=C(Me)-$ | NMe | Me | 4-Cl-Benzyl |
| 279 | $-CH_2ON=C(Me)-$ | NMe | Me | $MeOCH_2-$ |
| 280 | $-CH_2ON=C(Me)-$ | NMe | Me | $EtOCH_2-$ |

Table 15

| No. | A | B | $R^4$ | $R^5$ |
|---|---|---|---|---|
| 281 | -CH=NOCH(Me)- | NH | Me | Me |
| 282 | -CH=NOCH(Me)- | NH | Me | Et |
| 283 | -CH=NOCH(Me)- | NH | Me | n-Pr |
| 284 | -CH=NOCH(Me)- | NH | Me | i-Pr |
| 285 | -CH=NOCH(Me)- | NH | Me | Allyl |
| 286 | -CH=NOCH(Me)- | NH | Me | 2-Propynyl |
| 287 | -CH=NOCH(Me)- | NH | Me | Benzyl |
| 288 | -CH=NOCH(Me)- | NH | Me | 4-Cl-Benzyl |
| 289 | -CH=NOCH(Me)- | NH | Me | $MeOCH_2$- |
| 290 | -CH=NOCH(Me)- | NH | Me | $EtOCH_2$- |
| 291 | -CH=NOCH(Me)- | NMe | Me | Me |
| 292 | -CH=NOCH(Me)- | NMe | Me | Et |
| 293 | -CH=NOCH(Me)- | NMe | Me | n-Pr |
| 294 | -CH=NOCH(Me)- | NMe | Me | i-Pr |
| 295 | -CH=NOCH(Me)- | NMe | Me | Allyl |
| 296 | -CH=NOCH(Me)- | NMe | Me | 2-Propynyl |
| 297 | -CH=NOCH(Me)- | NMe | Me | Benzyl |
| 298 | -CH=NOCH(Me)- | NMe | Me | 4-Cl-Benzyl |
| 299 | -CH=NOCH(Me)- | NMe | Me | $MeOCH_2$- |
| 300 | -CH=NOCH(Me)- | NMe | Me | $EtOCH_2$- |

Table 16

| No. | A | B | $R^4$ | $R^5$ |
|---|---|---|---|---|
| 301 | -CH2ON=C(Me)- | O | Me | 4-Cl-α-Me-Benzyl |
| 302 | $-CH_2ON=C(Me)-$ | O | Me | c-Hexyl |
| 303 | $-CH_2ON=C(Me)-$ | O | Me | $Cl(CH_2)_2-$ |
| 304 | $-CH_2ON=C(Me)-$ | O | Me | $Cl(CH_2)_3-$ |
| 305 | $-CH_2ON=C(Me)-$ | O | Me | 3,4-$Cl_2$-Benzyl |
| 306 | -CH=NOCH(Me)- | O | Me | 4-Cl-α-Me-Benzyl |
| 307 | -CH=NOCH(Me)- | O | Me | c-Hexyl |
| 308 | -CH=NOCH(Me)- | O | Me | $Cl(CH_2)_2-$ |
| 309 | -CH=NOCH(Me)- | O | Me | $Cl(CH_2)_3-$ |
| 310 | -CH=NOCH(Me)- | O | Me | 3,4-$Cl_2$-Benzyl |
| 311 | $-CH_2ON=C(Et)-$ | O | Me | 4-Cl-α-Me-Benzyl |
| 312 | $-CH_2ON=C(Et)-$ | O | Me | c-Hexyl |
| 313 | $-CH_2ON=C(Et)-$ | O | Me | $Cl(CH_2)_2-$ |
| 314 | $-CH_2ON=C(Et)-$ | O | Me | $Cl(CH_2)_3-$ |
| 315 | $-CH_2ON=C(Et)-$ | O | Me | 3,4-$Cl_2$-Benzyl |
| 316 | -CH=NOCH(Et)- | O | Me | 4-Cl-α-Me-Benzyl |
| 317 | -CH=NOCH(Et)- | O | Me | c-Hexyl |
| 318 | -CH=NOCH(Et)- | O | Me | $Cl(CH_2)_2-$ |
| 319 | -CH=NOCH(Et)- | O | Me | $Cl(CH_2)_3-$ |
| 320 | -CH=NOCH(Et)- | O | Me | 3,4-$Cl_2$-Benzyl |

Table 17

| No. | A | B | $R^4$ | $R^5$ |
|---|---|---|---|---|
| 321 | $-CH_2ON=C(Me)-$ | O | H | Me |
| 322 | $-CH_2ON=C(Me)-$ | O | H | Et |
| 323 | $-CH_2ON=C(Me)-$ | O | H | n-Pr |
| 324 | $-CH_2ON=C(Me)-$ | O | H | i-Pr |
| 325 | $-CH_2ON=C(Me)-$ | O | H | Allyl |
| 326 | $-CH_2ON=C(Me)-$ | O | H | 2-Propynyl |
| 327 | $-CH_2ON=C(Me)-$ | O | H | Benzyl |
| 328 | $-CH_2ON=C(Me)-$ | O | H | 4-Cl-Benzyl |
| 329 | $-CH_2ON=C(Me)-$ | O | H | $MeOCH_2-$ |
| 330 | $-CH_2ON=C(Me)-$ | O | H | $EtOCH_2-$ |
| 331 | $-CH_2ON=C(Me)-$ | O | COOEt | Me |
| 332 | $-CH_2ON=C(Me)-$ | O | COOEt | Et |
| 333 | $-CH_2ON=C(Me)-$ | O | COOEt | n-Pr |
| 334 | $-CH_2ON=C(Me)-$ | O | COOEt | I-Pr |
| 335 | $-CH_2ON=C(Me)-$ | O | COOEt | Allyl |
| 336 | $-CH_2ON=C(Me)-$ | O | COOEt | 2-Propynyl |
| 337 | $-CH_2ON=C(Me)-$ | O | COOEt | Benzyl |
| 338 | $-CH_2ON=C(Me)-$ | O | COOEt | 4-Cl-Benzyl |
| 339 | $-CH_2ON=C(Me)-$ | O | COOEt | $MeOCH_2-$ |
| 340 | $-CH_2ON=C(Me)-$ | O | COOEt | $EtOCH_2-$ |

Table 18

| No. | A | B | $R^4$ | $R^5$ |
|---|---|---|---|---|
| 341 | $-CH=NOCH(Me)-$ | O | H | Me |
| 342 | $-CH=NOCH(Me)-$ | O | H | Et |
| 343 | $-CH=NOCH(Me)-$ | O | H | n-Pr |
| 344 | $-CH=NOCH(Me)-$ | O | H | i-Pr |
| 345 | $-CH=NOCH(Me)-$ | O | H | Allyl |
| 346 | $-CH=NOCH(Me)-$ | O | H | 2-Propynyl |
| 347 | $-CH=NOCH(Me)-$ | O | H | Benzyl |
| 348 | $-CH=NOCH(Me)-$ | O | H | 4-Cl-Benzyl |
| 349 | $-CH=NOCH(Me)-$ | O | H | $MeOCH_2-$ |
| 350 | $-CH=NOCH(Me)-$ | O | H | $EtOCH_2-$ |
| 351 | $-CH_2ON=C(Me)-$ | O | CN | Me |
| 352 | $-CH_2ON=C(Me)-$ | O | CN | Et |
| 353 | $-CH_2ON=C(Me)-$ | O | CN | n-Pr |
| 354 | $-CH_2ON=C(Me)-$ | O | CN | i-Pr |
| 355 | $-CH_2ON=C(Me)-$ | O | CN | Allyl |
| 356 | $-CH_2ON=C(Me)-$ | O | CN | 2-Propynyl |
| 357 | $-CH_2ON=C(Me)-$ | O | CN | Benzyl |
| 358 | $-CH_2ON=C(Me)-$ | O | CN | 4-Cl-Benzyl |
| 359 | $-CH_2ON=C(Me)-$ | O | CN | $MeOCH_2-$ |
| 360 | $-CH_2ON=C(Me)-$ | O | CN | $EtOCH_2-$ |

Table 19

| No. | A | B | $R^4$ | $R^5$ |
|---|---|---|---|---|
| 361 | $-CH=NOCH(Me)-$ | O | Ph | Me |
| 362 | $-CH=NOCH(Me)-$ | O | Ph | Et |
| 363 | $-CH=NOCH(Me)-$ | O | Ph | n-Pr |
| 364 | $-CH=NOCH(Me)-$ | O | Ph | i-Pr |
| 365 | $-CH=NOCH(Me)-$ | O | Ph | Allyl |
| 366 | $-CH=NOCH(Me)-$ | O | Ph | 2-Propynyl |
| 367 | $-CH=NOCH(Me)-$ | O | Ph | Benzyl |
| 368 | $-CH=NOCH(Me)-$ | O | Ph | 4-Cl-Benzyl |
| 369 | $-CH=NOCH(Me)-$ | O | Ph | $MeOCH_2-$ |
| 370 | $-CH=NOCH(Me)-$ | O | Ph | $EtOCH_2-$ |
| 371 | $-CH_2ON=C(Me)-$ | NPh | Me | Me |
| 372 | $-CH_2ON=C(Me)-$ | NPh | Me | Et |
| 373 | $-CH_2ON=C(Me)-$ | NPh | Me | n-Pr |
| 374 | $-CH_2ON=C(Me)-$ | NPh | Me | i-Pr |
| 375 | $-CH_2ON=C(Me)-$ | NPh | Me | Allyl |
| 376 | $-CH_2ON=C(Me)-$ | NPh | Me | 2-Propynyl |
| 377 | $-CH_2ON=C(Me)-$ | NPh | Me | Benzyl |
| 378 | $-CH_2ON=C(Me)-$ | NPh | Me | 4-Cl-Benzyl |
| 379 | $-CH_2ON=C(Me)-$ | NPh | Me | $MeOCH_2-$ |
| 380 | $-CH_2ON=C(Me)-$ | NPh | Me | $EtOCH_2-$ |

Table 20

| No. | A | B | $R^4$ | $R^5$ |
|---|---|---|---|---|
| 381 | -CH=NOCH(Et)- | O | Me | H |
| 382 | -CH=NOCH(Et)- | O | Me | Me |
| 383 | -CH=NOCH(Et)- | O | Me | Et |
| 384 | -CH=NOCH(Et)- | O | Me | n-Pr |
| 385 | -CH=NOCH(Et)- | O | Me | i-Pr |
| 386 | -CH=NOCH(Et)- | O | Me | n-Bu |
| 387 | -CH=NOCH(Et)- | O | Me | i-Bu |
| 388 | -CH=NOCH(Et)- | O | Me | s-Bu |
| 389 | -CH=NOCH(Et)- | O | Me | n-Pentyl |
| 390 | -CH=NOCH(Et)- | O | Me | n-Hexyl |
| 391 | -CH=NOCH(Et)- | O | Me | $CHF_2$ |
| 392 | -CH=NOCH(Et)- | O | Me | Allyl |
| 393 | -CH=NOCH(Et)- | O | Me | 2-Propynyl |
| 394 | -CH=NOCH(Et)- | O | Me | 2-Butenyl |
| 395 | -CH=NOCH(Et)- | O | Me | 2-Oxopropyl |
| 396 | -CH=NOCH(Et)- | O | Me | MeOC(=O)- |
| 397 | -CH=NOCH(Et)- | O | Me | EtOC(=O)- |
| 398 | -CH=NOCH(Et)- | O | Me | $EtOC(=O)CH_2$- |
| 399 | -CH=NOCH(Et)- | O | Me | EtOC(=O)CH(Me)- |
| 400 | -CH=NOCH(Et)- | O | Me | $MeOC(=O)CH_2$- |

Table 21

| No. | A | B | $R^4$ | $R^5$ |
|---|---|---|---|---|
| 401 | -CH=NOCH(Et)- | O | Me | Ph |
| 402 | -CH=NOCH(Et)- | O | Me | Benzyl |
| 403 | -CH=NOCH(Et)- | O | Me | α-Me-Benzyl |
| 404 | -CH=NOCH(Et)- | O | Me | 2-Me-Benzyl |
| 405 | -CH=NOCH(Et)- | O | Me | 3-Me-Benzyl |
| 406 | -CH=NOCH(Et)- | O | Me | 4-Me-Benzyl |
| 407 | -CH=NOCH(Et)- | O | Me | 2-Cl-Benzyl |
| 408 | -CH=NOCH(Et)- | O | Me | 3-Cl-Benzyl |
| 409 | -CH=NOCH(Et)- | O | Me | 4-Cl-Benzyl |
| 410 | -CH=NOCH(Et)- | O | Me | $MeOCH_2$- |
| 411 | -CH=NOCH(Et)- | O | Me | $EtOCH_2$- |
| 412 | -CH=NOCH(Et)- | O | Me | $MeO(CH_2)_2$- |
| 413 | -CH=NOCH(Et)- | O | Me | $EtO(CH_2)_2$- |
| 414 | -CH=NOCH(Et)- | O | Me | Acetyl |
| 415 | -CH=NOCH(Et)- | O | Me | $NCCH_2$- |
| 416 | -CH=NOCH(Et)- | O | Me | $NC(CH_2)_2$- |
| 417 | -CH=NOCH(Et)- | O | Me | c-Pentyl |
| 418 | -CH=NOCH(Et)- | O | Me | c-Pentyl$CH_2$- |
| 419 | -CH=NOCH(Et)- | O | Me | 5-Me-isoxazol -3-yl-methyl |
| 420 | -CH=NOCH(Et)- | O | Me | $CF_3CH_2$- |

Table 22

| No. | A | B | $R^4$ | $R^5$ |
|---|---|---|---|---|
| 421 | $-CH=NOC(Me)_2-$ | O | Me | Me |
| 422 | $-CH=NOC(Me)_2-$ | O | Me | Et |
| 423 | $-CH=NOC(Me)_2-$ | O | Me | n-Pr |
| 424 | $-CH=NOC(Me)_2-$ | O | Me | i-Pr |
| 425 | $-CH=NOC(Me)_2-$ | O | Me | Allyl |
| 426 | $-CH=NOC(Me)_2-$ | O | Me | 2-Propynyl |
| 427 | $-CH=NOC(Me)_2-$ | O | Me | Benzyl |
| 428 | $-CH=NOC(Me)_2-$ | O | Me | 4-Cl-Benzyl |
| 429 | $-CH=NOC(Me)_2-$ | O | Me | $MeOCH_2-$ |
| 430 | $-CH=NOC(Me)_2-$ | O | Me | $EtOCH_2-$ |
| 431 | -CH=NN=C(Et)- | O | Me | Me |
| 432 | -CH=NN=C(Et)- | O | Me | Et |
| 433 | -CH=NN=C(Et)- | O | Me | n-Pr |
| 434 | -CH=NN=C(Et)- | O | Me | i-Pr |
| 435 | -CH=NN=C(Et)- | O | Me | Allyl |
| 436 | -CH=NN=C(Et)- | O | Me | Benzyl |
| 438 | -CH=NN=C(Et)- | O | Me | 4-Cl-Benzyl |
| 439 | -CH=NN=C(Et)- | O | Me | $MeOCH_2-$ |
| 440 | -CH=NN=C(Et)- | O | Me | $EtOCH_2-$ |

Table 23

| No | Physical data |
|---|---|
| 1-1 | mp 109 to 110 ℃ |
| 1-2 | mp 125 to 126.5 ℃ |
| 1-5 | mp 97 to 98 ℃ |
| 1-12 | $^1$H-NMR($CDCl_3$) δ ppm : 1.85(3H, s), 1.93(3H, s), 4.00(3H, s), 4.60-4.63(2H, m), 5.09(2H, s), 5.17-5.32(2H, m), 5.92-6.06(1H, m), 6.74(1H, d, J=1.8), 7.24-7.51(4H, m), 8.37(1H, d,J=1.8). |
| 1-29 | mp 133.5 to 134.5 ℃ |
| 1-30 | mp 56 to 59 ℃ |
| 1-162 | $^1$H-NMR($CDCl_3$) δ ppm : 1.36(3H, d, J=6.7), 1.76(3H, s), 3.89(3H, s), 4.74(1H, q, J=6.7), 6.79(1H, d, J=1.2), 7.24-7.26(1H, m), 7.40-7.48(2H, m), 7.84-7.90(1H, m), 7.93(1H, s), 8.39(1H, d, J=1.2). |
| 1-211 | mp 122 to 123.5 ℃ |
| 1-221 | mp 135.5 to 136.5 ℃ |
| 1-231 | $^1$H-NMR($CDCl_3$) δ ppm : 1.84(3H, s), 3.80(3H, s), 3.96(3H, s), 3.99(3H, s), |
| | 5.07(2H, s), 6.74(1H, d, J=1.8), 7.22-7.48(4H, m), 8.38(1H, d, J=1.8). |
| 1-271 | $^1$H-NMR($CDCl_3$) δ ppm : 1.87(3H, s), 1.99(3H, s), 2.54(6H, s), 4.00(3H, s), |
| | 5.09(2H, s), 6.74(1H, d, J=1.8), 7.24-7.53(4H, m), 8.37(1H, d, J=1.8). |

Table 24

| No | Physical data |
|---|---|
| 3-2 | mp 99 to 100 ℃ |
| 4-2 | mp 78 to 79 ℃ |
| 5-2 | mp 83 to 84.5 ℃ |
| 6-2 | $^1$H-NMR($CDCl_3$) δ ppm : 1.90(3H, s), 1.91(3H, s), 3.93(3H, s), 4.04(3H, s), 5.10(2H, s), 7.03(1H, s), 7.12(1H, s), 7.34-7.50(4H, m), 8.00(1H, s). |
| 7-2 | mp 110 to 111 ℃ |
| 8-2 | $^1$H-NMR($CDCl_3$) δ ppm : 1.90(3H, s), 1.92(3H, s), 3.93(3H, s), 4.10(3H, s), 4.98(2H, s), 7.41-7.53(4H, m), 7.94(1H, s), 9.14(1H, s). |
| 9-2 | $^1$H-NMR($CDCl_3$) δ ppm : 1.87(3H, s), 1.90(3H, s), 3.92(3H, s), 3.98(3H, s), 5.10(2H, s), 6.11(1H, d, J=4.3), 6.36(1H, dd, J=3.7, 1.8), 7.22(1H, dd, J=7.3, 1.1.8), 7.35-7.53(4H, m). |
| 10-2 | mp 84 to 85 ℃ |
| 12-2 | mp 61 to 63 ℃ |
| 13-2 | mp 84 to 85 ℃ |

The following Test Examples illustrate the effects of the fungicide of this invention.

I. Controlling effect on various plant diseases by toliar treatment (pot experiment)

Experimental Method

A test compound was dissolved in a small amount of N,N-dimethylformamide, and the solution was diluted to a given concentration with distilled water containing a spreader. Thus, a liquid sample to be tested was prepared. The liquid sample was sprayed to test plants, and 24 hours thereafter, pathogens were inoculated by the method described below.

The percent control was calculated according to the following equation:

Percent control (%) =

$$\frac{\text{severity or number of lesion in untreated lot} - \text{severity or number of lesion in treated plot}}{\text{severity or number of lesion in untreated plot}} \times 100$$

Test example 1

Controlling effect on *Pyricularia oryzae*:

Two-week rice seedlings (var.: AICHIASAHI) were transplanted in plastic cups (each 9 cm in diameter) and cultivated another 2 weeks. The test compound in the form of a solution or a suspension was sprayed to the foliage of the rice seedlings, to which a conidia suspension of *Pyricularia oryzae* cultured in an oatmeal medium was inoculated by spraying. After the inoculation, the test plant was kept in a moist chamber (28 °C, 100 % R.H.) for 24 hours, followed by cultivation in a greenhouse for 5 days. Six days after inoculation, the number of lesions on the leaves of the inoculated plant was measured to calculate the percent control.

The results are shown as follows.

Controlling effect on *Pyricularia oryzae* by toliar treatment at 500 ppm

| Compound No. | (Percent control) |
|---|---|
| 1- 5 | 97 |
| 1- 12 | 90 |
| 1- 30 | 95 |
| 1- 162 | 90 |
| 1- 221 | 90 |
| 1- 231 | 90 |
| 1- 271 | 90 |
| 4- 2 | 97 |
| 7- 2 | 97 |
| 8- 2 | 97 |
| 9- 2 | 90 |
| 13- 2 | 97 |
| Control (Fthalide) | 97 |

Test Example 2

Controlling effect on *Sphaerotheca fuliginea*:

Seeds of cucumber (var.: TSUKUBASHIROIBO) were sown in plastic cups (each 9 cm in diameter), followed by cultivation for 2 to 3 weeks. The liquid test sample in the form of a solution or suspension was sprayed to the surface of their first leaves. The pathogen was inoculated to the leave by spraying a conidia suspension of *Sphaerotheca fuliginea* cultured on the cucumber leaves. After the inoculation, the plants were kept in a greenhouse at 20 °C for 10 days. Then, the infected area on the leaf was observed, and the percent control was calculated.

The results are shown as follows.

| Compound No. | Controlling effect on *Sphaerotheca fuliginea* by toliar treatment at 500 ppm (Percent control) |
|---|---|
| 1- 1 | 97 |
| 1- 2 | 100 |
| 1- 5 | 100 |
| 1- 12 | 100 |
| 1- 29 | 100 |
| 1- 30 | 100 |
| 1- 162 | 100 |
| 1- 221 | 100 |
| 1- 231 | 100 |
| 1- 271 | 100 |
| 3- 2 | 100 |
| 4- 2 | 100 |
| 5- 2 | 100 |
| 6- 2 | 100 |
| 7- 2 | 100 |
| 9- 2 | 100 |
| 12- 2 | 100 |
| 13- 2 | 100 |
| Control : Fenarimol | 100 |

Test Example 3

Controlling effect on *Botrytis cinerea*:

Seeds of cucumber (var.: TSUKUBASHIROIBO) were sown in plastic cups (each 9 cm in diameter), followed by cultivation for 2 to 3 weeks. The liquid test sample in the form of a solution or suspension was sprayed to the surface of their first leaves. The pathogen was inoculated by setting a hypha disk (∅ 4 mm) of *Botrytis cinerea* cultured in the

potato sucrose agar medium on the surface of the cucumber leave. After the inoculation, the plants were kept in a greenhouse at 20 °C for 3 days. Then, the diameter of lesions was observed, and the percent control was calculated.

The results are shown as follows.

| Compound No. | Controlling effect on *Botrytis cinerea* by toliar treatment at 500 ppm (Percent control) |
|---|---|
| 1- 12 | 70 |
| 1- 162 | 70 |
| 1- 231 | 70 |
| 4- 2 | 70 |
| 5- 2 | 70 |
| 6- 2 | 70 |
| 7- 2 | 70 |
| 12- 2 | 70 |
| 13- 2 | 70 |
| Control : Iprodione | 100 |

Test Example 4

Controlling effect on *Erysiphe graminis f. Sp. Tritici*:

The seeds of wheat (var.: NOURIN61GOU) were sown in plastic cups (each 9 cm in a diameter), followed by cultivation for 2 to 3 weeks. The test compound in the form of a solution or suspension was sprayed to the seedings, and conidia of *Erysiphe graminis f. Sp. tritici* cultured on the wheat leaves were dropped on the test plants to inoculate the plants with the pathogen. After the inoculation, the plants were kept in a greenhouse at 20 °C for 10 days. The infected area on the leaf was observed, and the percent control was calculated.

The results are shown as follows.

| Compound No. | Controlling effect on *Erysiphe graminis f. Sp. Tritici* by toliaa treatment at 500 ppm (Percent control) |
|---|---|
| 1- 2 | 97 |
| 1- 5 | 90 |
| 1- 12 | 97 |
| 1- 30 | 97 |
| 1- 162 | 90 |
| 1- 221 | 90 |
| 3- 2 | 90 |
| 4- 2 | 100 |
| 5- 2 | 97 |
| 7- 2 | 100 |
| 8- 2 | 90 |
| 10- 2 | 90 |
| 12- 2 | 90 |
| 13- 2 | 97 |
| Control: Fenarimol | 97 |

Industrial Applicability

The compounds of the present invention have a broad spectrum of fungicidal activity, and therefore, are useful as fungicides, particularly agricultural fungicides.

**Claims**

1. A compound represented by the formula (I):

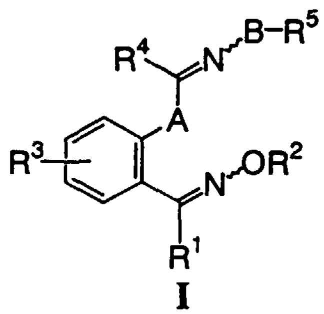

I

wherein $R^1$ is an optionally substituted heterocyclic group, CHO or $CH_2OR^6$ wherein $R^6$ is hydrogen atom, an alkyl group, an alkenyl group, an alkanoyl group, an alkoxycarbonyl group or an alkoxyalkyl group; $R^2$ is an alkyl group; $R^3$ is hydrogen atom, an alkyl group, an alkoxy group, a halogen atom, nitro, cyano or a halogen-substituted alkyl group; $R^4$ is hydrogen atom, an alkyl group, a cycloalkyl group, an alkoxyalkyl group, an alkoxycarbonyl group, cyano, a halogen-substituted alkyl group an optionally substituted aryl group or an optionally substituted heterocyclic group; $R^5$ is hydrogen atom, an optionally substituted alkyl group, a cycloalkyl group, an alkenyl group, an alkynyl group, an alkanoyl group, an aroyl group, an alkoxycarbonyl group, an optionally substituted aryl group or an optionally substituted heterocyclic group; A is (1) $-CH_2ON=C(R^7)-$ wherein $R^7$ is hydrogen atom, an alkyl group, a halogen-substituted alkyl group, cyano, an alkylthio group or a cycloalkyl group, (2) - $CH_2N(R^8)-N=C(R^7)-$ wherein $R^8$ is hydrogen atom, an alkyl group or an alkanoyl group and $R^7$ is the same as defined above, (3) - $CH=NOC(R^9)(R^{10})-$ wherein $R^9$ and $R^{10}$ are each independently hydrogen atom, an alkyl group, cyano or a halogen-substituted alkyl group or (4) $-CH=NN=C(R^7)-$ wherein $R^7$ is the same as defined above; B is oxygen atom or $NR^{11}$ wherein $R^{11}$ is hydrogen atom, an alkyl group, phenyl or an alkanoyl group; the wavy line: ~ indicates E isomer, Z isomer or a mixture of them.

2. A compound of claim 1 wherein said $R^1$ is pyridyl, pyrimidinyl, isoxazolyl, isoxazolinyl, isothiazolyl, thiadiazolyl, pyridadinyl, pyrazolyl, furyl, thienyl, imidazolyl, oxazolyl, thiazolyl, thiazolidinyl, oxadiazolyl, triazolyl or pyrazinyl, each of which may optionally be substituted, CHO or $CH_2OR^6$ wherein $R^6$ is the same as defined above.

3. A compound of claim 1 wherein said $R^1$ is isoxazol-3-yl, isoxazol-4-yl, isoxazol-5-yl, 2-isoxazolin-3-yl, 2-isoxazolin-5-yl, imdazol-1-yl, imdazol-2-yl, 1,3,4-oxadiazol-2-yl, furan-2-yl, 1,2,4-oxadiazol-3-yl, 1H-1,2,4-triazol-1-yl, thiazolidin-2-yl or oxazol-5-yl, each of which may optionally be substituted, CHO or $CH_2OR^6$ wherein $R^6$ is the same as defined above.

4. A compound of claim 1 wherein said $R^1$ is imidazol-1-yl, 1-methylimidazol-2-yl, isoxazol-3-yl, 3-methylisoxazol-5-yl, 5-methylisoxazol-3-yl, 2-isoxazolin-3-yl, 1,3,4-oxadiazol-2-yl, CHO, $CH_2OH$ or $CH_2OCH_3$.

5. A compound of claim 1 wherein said $R^2$ is methyl.

6. A compound of claim 1 wherein said $R^3$ is hydrogen atom.

7. A compound of claim 1 wherein said $R^4$ is hydrogen atom, methyl, ethyl, methoxycarbonyl, ethoxycarbonyl, cyano or an optionally substituted phenyl.

8. A compound of claim 1 wherein said $R^5$ is hydrogen atom, methyl, ethyl, propyl, isopropyl, butyl, allyl, 2-propynyl, methoxymethyl, ethoxymethyl, acetyl, methoxycarbonyl, ethoxycarbonyl or an optionally substituted benzyl.

9. A compound of claim 1 wherein said A is a group: $-CH_2ON=C(CH_3)-$, - $CH_2ON=C(C_2H_5)-$, $-CH_2ON=C(CN)-$, - $CH_2N(Ac)N=C(CH_3)-$, $-CH=NOCH(CH_3)-$, - $CH=NOCH(C_2H_5)-$, $-CH=NOC(CH_3)_2-$, $-CH=NN=C(CH_3)-$ or - $CH=NN=C(C_2H_5)-$.

10. A compound of claim 1 wherein said B is oxygen atom, -NH-, $-N(CH_3)-$, -N(Ph)- or N(Ac)-.

11. A compound of claim 1 which is

a compound wherein $R^1$ is isoxazol-3-yl, $R^2$ is methyl and $R^3$ is hydrogen atom,
a compound wherein $R^1$ is 2-isoxazolin-3-yl, $R^2$ is methyl and $R^3$ is hydrogen atom,
a compound wherein $R^1$ is 5-methylisoxazol-3-yl, $R^2$ is methyl and $R^3$ is hydrogen atom,
a compound wherein $R^1$ is 3-methylisoxazol-5-yl, $R^2$ is methyl and $R^3$ is hydrogen atom,
a compound wherein $R^1$ is imidazol-1-yl, $R^2$ is methyl and $R^3$ is hydrogen atom,
a compound wherein $R^1$ is thiazolidin-2-yl, $R^2$ is methyl and $R^3$ is hydrogen atom,
a compound wherein $R^1$ is 1,3,4-oxadiazol-2-yl, $R^2$ is methyl and $R^3$ is hydrogen atom,
a compound wherein $R^1$ is CHO, $R^2$ is methyl and $R^3$ is hydrogen atom,
a compound wherein $R^1$ is $CH_2OH$, $R^2$ is methyl and $R^3$ is hydrogen atom or
a compound wherein $R^1$ is $CH_2OCH_3$, $R^2$ is methyl and $R^3$ is hydrogen atom.

**12.** An agrochemical composition containing the compound of any one of claims 1 to 11 as an active ingredient.

**13.** A fungicidal composition containing the compound of any one of claims 1 to 11 as an active ingredient.

**INTERNATIONAL SEARCH REPORT**

International application No. PCT/JP97/01163

A. CLASSIFICATION OF SUBJECT MATTER Int. Cl[6] C07C251/50, 251/62, 251/74, 251/88, 255/63, C07D233/61, 249/08, 261/08, 271/10, 277/38, 307/52, C07D521/00, A01N37/50, 43/82, 43/88

According to International Patent Classification (IPC) or to both national classification and IPC

B. FIELDS SEARCHED

Minimum documentation searched (classification system followed by classification symbols) Int. Cl[6] C07C251/50, 251/62, 251/74, 251/88, 255/63, C07D233/61, 249/08, 261/08, 271/10, 277/38, 307/52, C07D521/00, A01N37/50, 43/82, 43/88

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| P,X | WO, 97/00866, A1 (CIBA-Geigy AG.), January 9, 1997 (09. 01. 97), Refer to claim (Family: none) | 1 - 13 |
| A | WO, 95/21156, A1 (BASF AG.), August 10, 1995 (10. 08. 95), Refer to claim & AU, 9514547, A & ZA, 9500859, A & EP, 741698, A1 & CZ, 9602317, A & NZ, 278588, A | 1 - 13 |
| A | WO, 95/21156, A2 (BASF AG.), August 10, 1995 (10. 08. 95), Refer to claim & AU, 9514160, A & WO, 95/21154, A3 & ZA, 9500860, A & EP, 741694, A1 & CZ, 9602315, A | 1 - 13 |
| A | WO, 95/21156, A1 (BASF AG.), August 10, 1995 (10. 08. 95), Refer to claim & AU, 9514546, A & ZA, 9500858, A & EP, 738259, A1 & CZ, 9602314, A | 1 - 13 |

[X] Further documents are listed in the continuation of Box C. [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| June 23, 1997 (23. 06. 97) | July 1, 1997 (01. 07. 97) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

| International application No. |
|---|
| PCT/JP97/01163 |

C (Continuation). DOCUMENTS CONSIDERED TO BE RELEVANT

| Category* | Citation of document, with indication, where appropriate, of the relevant passages | Relevant to claim No. |
|---|---|---|
| A | WO, 95/18789, A1 (CIBA-Geigy AG.), July 13, 1995 (13. 07. 95), Refer to claim & ZA, 9500027, A & AU, 9513851, A & BR, 9503066, A & NO, 9602823, A & FI, 9602712, A & EP, 738260, A1 & CZ, 9601990, A3 & SK, 9600880, A3 | 1 - 13 |

Form PCT/ISA/210 (continuation of second sheet) (July 1992)